# FASCICULE DE BREVET EUROPEEN

(11) **EP 2 070 988 B1**
(45) Date de publication et mention de la délivrance du brevet: **10.12.2014**
(21) Numéro de dépôt: 08164737.2
(22) Date de dépôt: 19.09.2008
(51) Int. Cl.: C09B 23/14, A61K 8/49, A61Q 5/10

(54) **Composé styryl à motif hydroxy(cyclo)alkylamino thiol/disulfure, procédé d'éclaircissement des matières kératiniques à partir de ce colorant**
Styrylverbindung mit einer Hydroxy(cyclo)alkylamino- sowie Thiol/Disulfideinheit, Verfahren zum Aufhellen keratinischer Materialien ausgehend von diesem Farbmittel
Styryl thiol/disulfide compound with a hydroxy(cyclo)alkylamino unit, process for lightening keratin materials using this dye

(30) Priorité: 21.09.2007 FR 0757755
(43) Date de publication de la demande: 17.06.2009
(73) Titulaire: L'Oréal, 75008 Paris (FR)
(72) Inventeur: Daubresse, Nicolas, 78170 La Celles Saint Cloud (FR); Greaves, Andrew, 77144 Montevrain (FR); Manfre, Franco, 94170 Le Perreux sur Marne (FR)
(74) Mandataire: Fevrier, Murielle Françoise E.

(56) Documents cités:
- EP-A- 1 647 580
- EP-A- 2 018 847
- WO-A-2005/097051
- WO-A-2006/134043
- WO-A-2006/136617
- WO-A-2007/025889
- WO-A-2007/110542
- OKAWA H ET AL: "SYNTHESIS AND CHARACTERIZATION OF AN ALKANETHIOL THIN FILM CONTAINING A HEMICYANINE DYE" MOLECULAR CRYSTALS AND LIQUID CRYSTALS SCIENCE AND TECHNOLOGY.SECTION A. MOLECULAR CRYSTALS AND LIQUID CRYSTALS, GORDON AND BREACH PUBLISHERS, CH, CH, vol. 377, 1 janvier 2002 (2002-01-01), pages 137-140, XP008056025 ISSN: 1058-725X

## Description

L'invention concerne la coloration de matières kératiniques à l'aide de colorants fluorescents styryl hydroxy(cyclo)alkylamino thiols/disulfures.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

La coloration des fibres kératiniques à partir de ces colorants directs classiques ne permet pas d'éclaircir de façon notable les fibres kératiniques.

L'éclaircissement de la couleur de fibres kératiniques foncées vers des nuances plus claires, en modifiant éventuellement la nuance de celles-ci, constitue une demande importante.

Classiquement, pour obtenir une coloration plus claire, on met en oeuvre un procédé de décoloration chimique. Ce procédé consiste à traiter les fibres kératiniques, telles que les cheveux, par un système oxydant fort, généralement constitué par du peroxyde d'hydrogène associé ou non à des persels, le plus souvent en milieu alcalin.

Ce système de décoloration présente l'inconvénient de dégrader les fibres kératiniques, et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres, particulièrement dans les traitements de défrisage.

Une autre technique d'éclaircissement consiste à appliquer sur les cheveux foncés des colorants directs fluorescents. Cette technique décrite notamment dans les documents WO 03/028685, WO 2004/091473 permet de respecter la qualité de la fibre kératinique lors du traitement. Cependant ces colorants directs fluorescents ne possèdent pas une tenacité satisfaisante vis-à-vis des agents extérieurs.

Pour augmenter la ténacité des colorations directes, il est connu d'utiliser des colorants disulfures notamment colorants à chromophore aza-imidazoliums dans les demandes de brevet WO 2005/097051 ou EP 1647580, et des colorants à chromophores pyridinium/indolinium-styryles dans les demandes de brevet WO 2006/134043, WO 2006/136617et WO 2007/025889.

Le but de la présente invention est de fournir de nouveaux systèmes de coloration de matières kératiniques en particulier les fibres kératiniques humaines, notamment les cheveux, qui ne présentent pas les inconvénients des procédés de décoloration existants.

Particulièrement, un but de l'invention est de fournir des systèmes de coloration directe permettant d'obtenir des effets éclaircissants notamment sur des fibres kératiniques naturellement ou artificiellement foncées, tenaces face à des shampooings successifs, qui ne dégradent pas les fibres kératiniques et qui n'altèrent pas leurs propriétés cosmétiques.

Un autre but de l'invention est de colorer de façon chromatique et rémanente vis-à-vis des agressions extérieures les matières kératiniques. L'invention vise également à mettre à disposition des composés colorant les fibres kératiniques telles que les cheveux avec une faible sélectivité de coloration entre la racine et la pointe, que ce soit sur fibres naturelles ou permanentées.

Ces buts sont atteints avec la présente invention qui a pour objet un procédé de coloration de matières kératiniques, en particulier les fibres kératiniques, notamment humaines telles que les cheveux, plus particulièrement les cheveux foncés, consistant à appliquer sur les matières kératiniques, une composition tinctoriale, comprenant dans un milieu cosmétique approprié, au moins un colorant fluorescent disulfure ou thiol, choisi parmi les colorants de formule **(I)** : 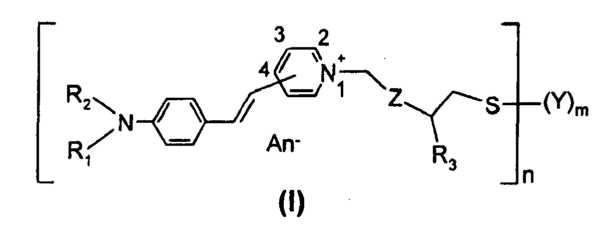 leurs sels d'acide organique ou minéral, isomères optiques, isomères géométriques, et les solvates tels que hydrates ; formule **(I)** dans laquelle :
- **R₁**, représente un groupe alkyle en C₁-C₆ substitué par un ou plusieurs groupes hydroxyle ou -C(O)OR' avec R' représentant un atome d'hydrogène, un groupe alkyle en C₁-C₄ ou alors un groupe -C(O)-O⁻ et dans ce dernier cas un contre ion anionique An⁻ est absent, particulièrement R₁ représente un groupe alkyle en C₁-C₆ substitué par ou plusieurs groupes hydroxyle et plus spécifiquement par un seul groupe hydroxyle ;
- **R₂**, représente un groupe alkyle en C₁-C₆ éventuellement substitué par un ou plusieurs groupes hydroxyle ;
- ou alors les groupements R₁ et R₂ forment ensemble avec l'atome d'azote qui les portent un radical hétérocyclique saturé substitué au moins par un groupement hydroxyle, (poly)hydroxy(C₁-C₄)alkyle, et/ou -C(O)OR' avec R' représentant un atome d'hydrogène, un groupe alkyle en C₁-C₄ ou alors un groupe -C(O)-O- et dans ce cas un contre ion anionique An⁻ est absent, ; tel que pyrrolidinyle et pipéridyle ;
- **R₃** représente un atome d'hydrogène ou un groupement -C(O)OR" avec R" représentant un atome d'hydrogène, un métal alcalin, un groupement alkyle en C₁-C₆ ou alors R₃ représente un groupe -C(O)-O- et dans ce cas un contre-ion anionique An⁻ est absent ;
- **Z** représente un groupe divalent amido -C(O)-N(R)-, -N(R)-C(O)-, ou un groupe divalent alkylène en C₁-C₁₀ interrompu par un groupe amido -C(O)-N(R)-, -N(R)-C(O)-tel que -(CH₂)_{n'}-C(O)-N(R)-(CH₂)ₚ-, -(CH₂)_{n"}-N(R)-C(O)-(CH₂)ₚ-, avec n' représentant un entier compris inclusivement entre 0 et 3, préférentiellement n' vaut 0, 2, 3 ; p représentant un entier compris inclusivement entre 0 et 4, n" représentant un entier compris inclusivement entre 0 et 3 et notamment n'=n"=p=0 et R représentant un atome d'hydrogène ou un groupe alkyle en C₁-C₆ ;
- **n** vaut 1 ou 2 ;
- **m** vaut 0 ou 1 ;
- **An⁻** représente un contre ion anionique ;
- **Y** représente : i) un atome d'hydrogène, ii) un métal alcalin, iii) un métal alcalino-terreux, iv) un groupe ammonium : N⁺R^{α}R^{β}R^{γ}R^{δ}, un groupe phosphonium : P⁺R^{α}R^{β}R^{γ}R^{δ} avec R^{α}, R^{β}, R^{γ} et R^{δ}, identiques ou différents, représentant un atome d'hydrogène ou un groupe (C₁-C₄)alkyle, ou v) un groupe protecteur de fonction thiol ; étant entendu que :
   - la liaison entre le cycle pyridinium et la double liaison du groupe styryle est positionnée en position 2 ou 4 du pyridinium ;
   - lorsque n=1, m=1 alors Y représente i) à v) telque défini précédemment et lorsque n=2, alors m=0
   - lorsque le composé de formule **(I)** contient d'autres parties cationiques, il se trouve associé à un ou plusieurs contre-ions anioniques permettant d'atteindre l'électroneutralité de la formule **(I)**; et étant entendu que le composé de formule (I) est différents des composés suivants : 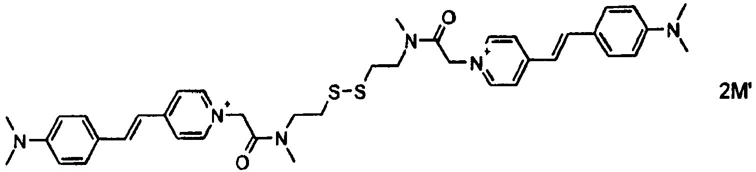 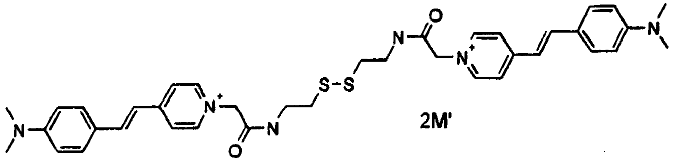 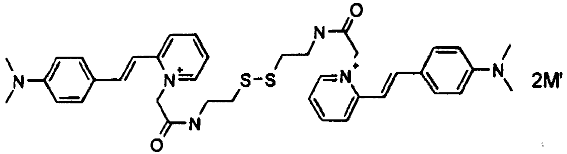 avec M'un contre-ion anionique.

Un autre objet de l'invention est une composition tinctoriale comprenant, dans un milieu cosmétique approprié au moins un colorant fluorescent thiol/disulfure **(I)** tel que défini précédemment, et éventuellement un agent réducteur.

L'invention a aussi pour objet de nouveaux colorants fluorescents thiols/disulfures de formule **(I)**.

Le procédé de coloration selon l'invention permet de colorer de façon visible les matières kératiniques foncées, en particulier les fibres kératiniques humaines foncées, notamment les cheveux foncés.

De plus le procédé de l'invention permet d'obtenir une coloration des matières kératiniques en particulier les fibres kératiniques humaines, notamment les cheveux, sans dégrader ladite matière, rémanente vis-à-vis de shampooings, des agressions courantes (soleil, transpiration), et des autres traitements capillaires. Le procédé de l'invention permet également d'obtenir un éclaircissement des matières kératiniques telles que les fibres kératiniques particulièrement les fibres kératiniques foncées et plus particulièrement les cheveux foncés.

Les nouveaux colorants selon l'invention présentent une photostabilité et une stabilité chimique très satisfaisantes. Ces colorants sont solubles dans les milieux cosmétiques appropriés aux teintures capillaires et tout particulièrement dans les mélanges eau/éthanol. Ils permettent d'étendre la gamme colorielle accessible (colorants jaunes, oranges, rouges) tout en laissant la possibilité de décaper les colorations obtenues.

Les colorants de formule **(I)** colorent après application sur fibres kératiniques de façon chromatique et rémanente vis-à-vis des agressions extérieures les matières kératiniques avec une faible sélectivité de coloration entre la racine et la pointe, et sur différents types de fibres.

Au sens de l'invention, on entend par matière kératinique foncée celle qui présente une luminescence L* chiffrée dans le système C.I.E. L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la variation de «hauteur de ton» avant et après application du composé de formule **(I)**. La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitements capillaires » de Charles ZVIAK 1988, Ed. Masson, p. 215 et 278.

Les hauteurs de ton s'échelonnent de 1 (noir) à 10 (blond très clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

Au sens de l'invention, on entend par cheveux décolorés, des cheveux dont la hauteur de ton est supérieure à 6 et de préférence supérieure à 7.

Au sens de l'invention on entend par « décapage » de la coloration des fibres kératiniques un procédé qui fait intervenir l'application d'un stimulus chimique ou physique apte à ramener le cheveu vers sa couleur originelle. A titre d'exemple le décapage peut être obtenu par application d'une composition oxydante à base de peroxyde d'hydrogène, de persulfate de sodium, de potassium ou d'ammonium, à un pH modérément alcalin, *i.e.* compris entre 7 et 12, préférentiellement entre 8 et 10,5.

Un moyen pour mesurer l'effet éclaircissant apporté aux cheveux après application des colorants fluorescent de l'invention est d'utiliser le phénomène de réflectance des cheveux.

De préférence, la composition doit, après application sur des cheveux foncés amener aux résultats ci-dessous.
- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 500 à 700 nanomètres supérieure à la courbe correspondant aux cheveux non traités.
- Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Au sens de la présente invention, et à moins qu'une indication différente ne soit donnée :
- les radicaux « aryle » ou « hétéroaryle » ou la partie aryle ou hétéroaryle d'un radical peuvent être substitués par au moins un substituant porté par un atome de carbone, choisi parmi :
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyle, identiques ou différents, en C₁-C₄, éventuellement porteurs d'au moins un groupe hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
   - un atome d'halogène tel que chlore, fluor ou brome ;
   - un groupe hydroxy ;
   - un radical alcoxy en C₁-C₂ ;
   - un radical alkylthio en C₁-C₂ ;
   - un radical (poly)-hydroxyalcoxy en C₂-C₄ ;
   - un radical amino ;
   - un radical héterocycloalkyle à 5 ou 6 chaînons ;
   - un radical hétéroaryle à 5 ou 6 chaînons éventuellement cationique, préférentiellement imidazolium, et éventuellement substitué par un radical (C₁-C₄) alkyle, préférentiellement méthyle ;
   - un radical amino substitué par un ou deux radicaux alkyle, identiques ou différents, en C₁-C₆ éventuellement porteurs d'au moins :
      i) un groupe hydroxy,
      ii) un groupe amino éventuellement substituté par un ou deux radicaux alkyle en C₁-C₃ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
   - -NR-COR' dans lequel le radical R est un atome d'hydrogène, un radical alkyle en C₁-C₄ éventuellement porteur d'au moins un groupe hydroxy et le radical R' est un radical alkyle en C₁-C₂ ;
   - (R)₂N-CO- dans lequel les radicaux R, identiques ou non, représentent un atome d'hydrogène, un radical alkyle en C₁-C₄ éventuellement porteur d'au moins un groupe hydroxy ;
   - R'SO₂-NR- dans lequel le radical R représente un atome d'hydrogène, un radical alkyle en C₁-C₄ éventuellement porteur d'au moins un groupe hydroxy et le radical R' représente un radical alkyle en C₁-C₄, un radical phényle ;
   - (R)₂N-SO₂- dans lequel les radicaux R, identiques ou non, représentent un atome d'hydrogène, un radical alkyle en C₁-C₄ éventuellement porteur d'au moins un groupe hydroxy,
   - un radical carboxylique sous forme acide ou salifiée (de préférence avec un métal alcalin ou un ammonium, substitué ou non) ;
   - un groupe cyano ;
   - un groupe polyhalogénoalkyle comprenant de 1 à 6 atomes de carbones et de 1 à 6 atome d'halogène, identiques ou différents, le groupe polyhalogénoalkyle est par exemple le trifluorométhyle ;
- la partie cyclique ou hétérocyclique d'un radical non aromatique peut être substituée par au moins un substituant porté par un atome de carbone choisi parmi les groupes :
   - hydroxy,
   - alcoxy en C₁-C₄,
   - (poly)hydroxyalcoxy en C₂-C₄,
   - un radical alkylthio en C₁-C₂ ;
   - RCO-NR'- dans lequel le radical R' est un atome d'hydrogène, un radical alkyle en C₁-C₄ éventuellement porteur d'au moins un groupe hydroxy et le radical R est un radical alkyle en C₁-C₂, amino substitué par deux groupes alkyle identiques ou différents en C₁-C₄ éventuellement porteurs d'au moins un groupe hydroxy ;
   - RCO-O- dans lequel le radical R est un radical alkyle en C₁-C₄, amino substitué par un ou deux groupes alkyle identiques ou différents en C₁-C₄ éventuellement porteurs d'au moins un groupe hydroxy, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - RO-CO- dans lequel le radical R est un radical alkyle en C₁-C₄ éventuellement porteurs d'au moins un groupe hydroxy ;
- un radical cyclique, hétérocyclique, ou une partie non aromatique d'un radical aryle ou hétéroaryle, peut également être substitué par un ou plusieurs groupes oxo ou thioxo ;
- un radical « aryle » représente un groupe carboné mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un radical « diarylalkyle » représente un groupe comportant sur le même atome de carbone d'un groupe alkyle deux groupe aryle, identiques ou différents tel que diphénylméthyle ou 1,1-diphényléthyle ;
- un « radical hétéroaryle » représente un groupe mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tétrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un radical « dihétéroarylalkyle » représente un groupe comportant sur le même atome de carbone d'un groupe alkyle deux groupe hétéroaryle, identiques ou différents tel que difurylméthyle, 1,1-difuryléthyle, dipyrrolylméthyle, dithiénylméthyle ;
- un « radical cyclique » est un radical cycloalkyle non aromatique, mono ou polycyclique, condensé ou non, contenant de 5 à 22 atomes de carbone, pouvant comporter de 1 à plusieurs insaturations ; particulièrement le radical cyclique est un cyclohexyle ;
- un radical « cyclique stériquement encombré» est un radical cyclique, aromatique ou non, substitué ou non, encombré par effet ou contrainte stérique, comprenant de 6 à 14 chaînons, pouvant être pontés, à titre de radicaux stériquement encombrés on peut citer le bicyclo[1.1.0]butane, les mésytyles tels que le 1,3,5-triméthylpnényle, le 1,3,5-triterbutylphényle, le 1,3,5-isobutylphényle, le 1,3,5-trimétylsillylphényle et l'adamantyle ;
- un « radical hétérocyclique ou hétérocycle» est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium, particulièrement le radical est monocyclique, contient de 5 à 7 chaînons et 1 ou 2 hétéroatomes choisis parmi l'azote, l'oxygène et le soufre, dont au moins un des hétéroatome est un atome d'azote ; plus particulièrement le radical hétérocyclique est choisi parmi pyrrolidinyle, et pipéridyle ;
- un « radical alkyle » est un radical hydrocarboné en C₁-C₁₆, linéaire ou ramifié, de préférence en C₁-C₈ ;
- l'expression « éventuellement substitué » attribué au radical alkyle sous entend que ledit radical alkyle peut être substitué par un ou plusieurs radicaux choisis parmi les radicaux i) hydroxy, ii) alcoxy en C₁-C₄, iii) acylamino, iv) amino éventuellement substitué par un ou deux radicaux alkyle, identiques ou différents, en C₁-C₄, lesdits radicaux alkyles pouvant former avec l'atome d'azote qui les portent un hétérocyle comprenant de 5 à 7 chaînons, comprenant éventuellement un autre hétéroatome différent ou non de l'azote ; v) ou un groupe ammonium quaternaire -N⁺R'R''R''', M⁻pour lequel R', R", R"', identiques ou différents représentent un atome d'hydrogène, ou un groupe alkyle en C₁-C₄, ou alors -N⁺R'R"R"' forme un hétéroaryle tel que imidazolium éventuellement substitué par un groupe C₁-C₄ alkyle, et M représente le contre-ion de l'acide organique, minéral ou de l'halogénure correspondant,
- un « radical alcoxy » est un radical alkyle-oxy ou alkyl-O- pour lequel le radical alkyle est un radical hydrocarboné, linéaire ou ramifié, en C₁-C₁₆ préférentiellement en C₁-C₈ ;
- un « radical alkylthio » est un radical alkyl-S- pour lequel le radical alkyle est un radical hydrocarboné, linéaire ou ramifié, en C₁-C₁₆ préférentiellement en C₁-C₈ ; lorsque le groupe alkylthio est éventuellement substitué, cela sous entend que le groupe alkyle est éventuellement substitué tel que défini précédemment ;
- les bornes délimitant l'étendue d'une plage de valeurs sont comprises dans cette plage de valeurs ;
- un « sel d'acide organique ou minéral » est plus particulièrement choisi parmi un sel dérivé i) d'acide chlorhydrique HCl, ii) d'acide bromhydrique HBr, iii) d'acide sulfurique H₂SO₄, iv) d'acides alkylsulfoniques : Alk-S(O)₂OH tels que d'acide méthylsulfonique et d'acide éthylsulfonique ; v) d'acides arylsulfoniques : Ar-S(O)₂OH tel que d'acide benzène sulfonique et d'acide toluène sulfonique ; vi) d'acide citrique ; vii) d'acide succinique ; viii) d'acide tartrique ; ix) d'acide lactique, x) d'acides alkoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique H₃PO₄; xiii) d'acide acétique CH₃COOH ; xiv) d'acide triflique CF₃SO₃H et xv) d'acide tétrafluoroborique HBF₄ ;
- un « contre-ion anionique » est un anion ou un groupe anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les C₁-C₆ alkylsulfonates : Alk-S(O)₂O⁻ tels que le méthylsulfonate ou mésylate et l'éthylsulfonate ; iv) les arylsulfonates : Ar-S(O)₂O⁻ tel que le benzènesulfonate et le toluènesulfonate ou tosylate ; v) le citrate ; vi) le succinate ; vii) le tartrate ; viii) le lactate ; ix) les alkylsulfates : Alk-O-S(O)O⁻ tels que le méthysulfate et l'éthylsulfate ; x) les arylsulfates : Ar-O-S(O)O⁻ tels que le benzènesulfate et le toluènesulfate ; xi) les alkoxysulfates : Alk-O-S(O)₂O⁻ tel que le méthoxy sulfate et l'éthoxysulfate ; xii) les aryloxysulfates : Ar-O-S(O)₂O⁻, xiii) le phosphate ; xiv) l'acétate ; xv) le triflate ; et xvi) les borates tels que le tétrafluoroborate ;
- les « solvates » représentent les hydrates ainsi que l'association avec des alcools linéaires ou ramifiés en C1-C4 linéaire ou ramifié tels que l'éthanol, l'isopropanol, le n-propanol.

Un des objets de l'invention concernent des colorants fluorescents thiols/disulfure de formule **(I)**.

Les colorants fluorescents thiols/disulfures de formule **(I)** sont des composés capables d'absorber dans le rayonnement UV ou visible à une longueur d'onde λ_{abs} comprise entre 250 et 800 nm et capables de réémettre dans le domaine du visible à une longueur d'onde d'émission λ_{ém} comprise entre 400 et 800 nm.

De préférence les composés fluorescents de formule **(I)** de l'invention sont des colorants capables d'absorber dans le visible λ_{abs} comprise entre 400 et 800 nm et de réémettre dans le visible λ_{ém} comprise entre 400 et 800 nm. Plus préférentiellement les colorants de formule **(I)** sont des colorants capables d'absorber à une λ_{abs} comprise entre 420 nm et 550 nm et de réémettre dans le visible à une λ_{ém} comprise entre 470 et 600 nm.

Les composés fluorescents thiol de l'invention de formule **(I)** pour lesquels m et n valent 1, contiennent une fonction SY qui peut se trouver sous la forme covalente -S-Y ou ionique -S⁻Y⁺ selon la nature de Y et du pH du milieu.

Lorsque les composés de l'invention contiennent plusieurs contre-ions anioniques An⁻, lesdits contre-ions peuvent être identiques ou différents.

Un mode particulier concerne les colorants fluorescents thiols de formule **(II)** pour lesquels m et n valent 1, à fonction SY où Y représente un atome d'hydrogène, ou un métal alcalin. Avantageusement Y représente un atome d'hydrogène.

Conformément à un autre mode de réalisation particulier de l'invention, dans la formule **(I)** précitée, Y est un groupe protecteur connu par l'homme du métier comme par exemple ceux décris dans les ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5. Etant entendu que Y, en tant que groupe protecteur, ne peut pas constituer avec l'atome de soufre sur lequel il est attaché un colorant disulfure i.e. ne peut pas constituer une formule (I) dans laquelle m=2 et n=0. Y en tant que groupe protecteur ne peut pas représenter un groupe directement attaché à l'atome de soufre de formule (I) via un autre atome de soufre non oxydé.

Particulièrement lorsque Y représente un groupe protecteur de la fonction thiol, Y est choisi parmi les radicaux suivants :
■ (C₁-C₄)alkylcarbonyle ;
■ (C₁-C₄)alkylthiocarbonyle ;
■ (C₁-C₄)alcoxycarbonyle ;
■ (C₁-C₄)alcoxythiocarbonyle ;
■ (C₁-C₄)alkylthio-thiocarbonyle ;
■ (di)(C₁-C₄)(alkyl)aminocarbonyle ;
■ (di)(C₁-C₄)(alkyl)aminothiocarbonyle;
■ arylcarbonyle comme phénylcarbonyle ;
■ aryloxycarbonyle ;
■ aryl(C₁-C₄)alkcoxycarbonyle ;
■ (di)(C₁-C₄)(alkyl)aminocarbonyle comme diméthylaminocarbonyle ;
■ (C₁-C₄)(alkyl)arylaminocarbonyle ;
■ carboxy ;
■ SO₃⁻; M⁺ avec M⁺ représentant un métal alcalin tel que le sodium ou le potassium, ou alors An⁻ de la formule (I) et M⁺ sont absents ;
■ aryle éventuellement substitué tel que le phényle, dibenzosubéryle, ou 1,3,5-cycloheptatriényle,
■ hétéroaryle éventuellement substitué ; dont notamment l'hétéroaryle cationiques ou non, comprenant de 1 à 4 hétéroatomes suivants :
   i) monocycliques à 5, 6 ou 7 chaînons tels que furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;
   ii) bicycliques à 8 à 11 chaînons tels que indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par un ou plusieurs groupes tels que (C₁-C₄)alkyle comme méthyle, ou polyhalogéno(C₁-C₄)alkyle comme trifluorométhyle ;
   iii) ou tricyclique ABC suivant : 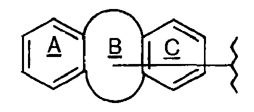
dans lequel les deux cycles A, C comportent éventuellement un hétéroatome, et le cycle B est un cycle à 5, 6 ou 7 chaînons particulièrement à 6 chaînons et contient au moins un hétéroatome comme pypéridyle, pyranyle ;
■ hétérocycloalkyle éventuellement substitué, éventuellement cationique, le groupe hétérocycloalkyle représente notamment un groupe monocyclique saturé ou partiellement saturé à 5, 6 ou 7 chaînons comprenant de 1 à 4 hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexahydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme (C₁-C₄)alkyle, oxo ou thioxo ; ou l'hétérocycle représente le groupe suivant : 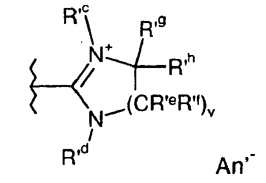
dans lequel R'^{c}, R'^{d}, R'^{e}, R'^{f}, R'^{g} et R'^{h}, identiques ou différents représentent un atome d'hydrogène ou un groupe (C₁-C₄)alkyle, ou alors deux groupe R'^{g} avec R'^{h}, et/ou R'^{e} avec R'^{f} forment un groupe oxo ou thioxo, ou alors R'^{g} avec R'^{e} forment ensemble un cycloalkyle ; et v représente un entier compris inclusivement entre 1 et 3 ; préférentiellement R'^{c} à R'^{h} représentent un atome d'hydrogène ; et An'⁻ représente un contre-ion anionique;
▪ isothiouronium ;
▪ -C(NR'^{c}R'^{d})=N⁺R'^{e}R'^{f}; An⁻ avec R'^{c}, R'^{d}, R'^{e} et R'^{f}, identiques ou différents représentent un atome d'hydrogène ou un groupe (C₁-C₄)alkyle ; préférentiellement R'^{c} à R'^{f} représentent un atome d'hydrogène ; et An'⁻représente un contre-ion anionique;
▪ isothiourée ;
▪ -C(NR'^{c}R'^{d})=NR'^{e}; An⁻ avec R'^{c}, R'^{d}, R'^{e} et An⁻ sont tels que définis précédemment ;
▪ (di)aryl(C₁-C₄)alkyle éventuellement substitué tel que le 9-anthracénylméthyle, phénylméthyle ou diphénylméthyle éventuellement substitué par un plusieurs groupes notamment choisis parmi (C₁-C₄) alkyle, (C₁-C₄) alcoxy comme le méthoxy, hydroxy, alkylcarbonyle, (di)(C₁-C₄)(alkyl)amino comme le diméthylamino ;
▪ (di)hétéroaryl(C₁-C₄)akyle éventuellement substitué, le groupe hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupe tel que alkyle particulièrement méthyle, avantageusement le (di)hétéroaryl(C₁-C₄)akyle est (di)hétéroarylméthyle ou (di)hétéroaryléthyle ;
▪ -CR¹R²R³ avec R¹, R² et R³ identiques ou différents, représentant un atome d'halogène ou un groupe choisi parmi :
   i) (C₁-C₄)alkyle ;
   ii) (C₁-C₄)alcoxy ;
   iii) aryle éventuellement substitué tel que phényle éventuellement substitué par un ou plusieurs groupes comme (C₁-C₄)alkyle, (C₁-C₄)alcoxy, hydroxy ;
   iv) hétéroaryle éventuellement substitué tel que thiophényle, furanyle, pyrrolyle, pyranyle, pyridyle, éventuellement substitué par un groupe (C₁-C₄)alkyle ;
   v) P(Z¹)R'¹R'²R'³ avec R'¹, et R'² identiques ou différents représentent un groupe hydroxy, (C₁-C₄)alcoxy ou alkyle, R'³ représente un groupe hydroxy ou (C₁-C₄)alcoxy, et Z¹ représente un atome d'oxygène ou de soufre ;
▪ cyclique stériquement encombré ; et
▪ alcoxyalkyle éventuellement substitué tels que le méthoxyméthyle (MOM), éthoxyéthyle (EOM) et l'isobutoxyméthyle.

Selon un mode de réalisation particulier les colorants fluorescents thiols protégés de formule **(I)** pour lesquels m et n valent 1 comportent un groupe Y i) hétéroaryle monocyclique à 5 ou 6 chaînons aromatiques, cationiques comprenant de 1 à 4 hétéroatomes choisis parmi l'oxygène, le soufre et l'azote, tels que oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupes tels que alkyle comme méthyle, ou polyhalogéno(C₁-C₄)alkyle comme trifluorométhyle ; iii) ou hétérocyclique suivant : 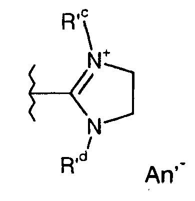 dans lequel R'^{c} et R'^{d}, identiques ou différents, représentent un atome d'hydrogène ou un groupe (C₁-C₄)alkyle ; préférentiellement R'^{c} à R'^{d} représentent un groupe (C₁-C₄)alkyle tel que méthyle ; et An'⁻ représente un contre-ion anionique.

Particulièrement Y représente un groupe choisi parmi oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium et imidazolium, benzoimidazolium, benzoxazolium, benzothiazolium, ces groupes étant éventuellement substitués par un ou plusieurs groupes (C₁-C₄) alkyle notamment méthyle. En particulier Y représente un métal alcalin ou un groupe protecteur tel que:
➢ (C₁-C₄)alkylcarbonyle comme méthylcarbonyle ou éthylcarbonyle ;
➢ arylcarbonyle comme phénylcarbonyle ;
➢ (C₁-C₄)alkoxycarbonyle ;
➢ aryloxycarbonyle ;
➢ aryl(C₁-C₄)alkoxycarbonyle ;
➢ (di)(C₁-C₄)(alkyl)aminocarbonyle comme diméthylaminocarbonyle;
➢ (C₁-C₄)(alkyl)arylaminocarbonyle ;
➢ aryle éventuellement substitué tel que le phényle ;
➢ hétéroaryle monocyclique à 5 ou 6 chaînons tels que imidazolyle ou pyridyle ;
➢ hétéroaryle monocyclique cationique à 5, 6 chaînons tels que pyrylium, pyridinium, pyrimidinium, pyrazinium, pyridazinium, triazinium, imidazolium ; ces groupes étant éventuellement substitués par un ou plusieurs, identiques ou différents, groupes (C₁-C₄)alkyle tel que méthyle ;
➢ hétéroaryle bicyclique cationique à 8 à 11 chaînons tels que benzoimidazolium, ou le benzoxazolium ; ces groupes étant éventuellement substitué par un ou plusieurs, identiques ou différents, groupes (C₁-C₄)alkyle tel que méthyle ;
➢ hétérocycle cationique de formule suivante : 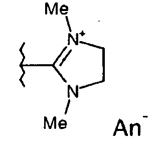
➢ isothiouronium -C(NH₂)=N⁺H₂; An⁻;
➢ isothiourée -C(NH₂)=NH ;
➢ SO₃⁻, M⁺ avec M⁺ représentant un métal alcalin tel que le sodium ou le potassium, ou alors An⁻ de la formule (I) et M⁺ sont absents.

Conformément à un mode de réalisation particulier de l'invention les colorants fluorescents disulfures de formule **(I)** lorsque n vaut 2 et m vaut 0 présentent un axe de symétrie C2 entre les deux atomes de soufre du radical central disulfure.

Selon un mode particulier de l'invention, les colorants de l'invention appartiennent à une des deux formules **(Ia)** ou **(Ib)** suivantes : 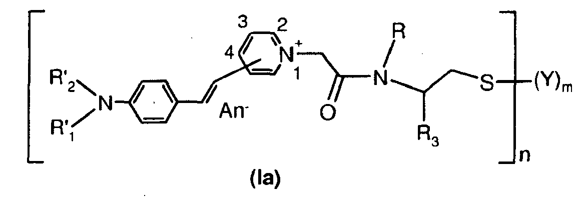 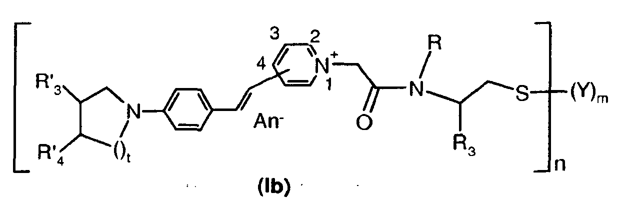 leurs sels d'acide organique ou minéral, isomères optiques, isomères géométriques, et les solvates tels que hydrates ; formules **(Ia)** et **(Ib)** avec :
- **R'₁**, représente un groupe alkyle en C₁-C₄ substitué par un ou plusieurs groupes hydroxyle, particulièrement par un seul groupe hydroxyle ou -C(O)OR' avec R' représentant un atome d'hydrogène, un groupe alkyle en C₁-C₄ ou alors un groupe - C(O)-O⁻ et dans ce dernier cas un contre ion anionique An⁻ est absent;
- **R'₂**, représente un groupe alkyle en C₁-C₄ éventuellement substitué par un ou plusieurs groupes hydroxyle ; particulièrement par un seul groupe hydroxyle ; plus particulièrement R'₁ et R'₂ sont identiques ;
- **R'₃**, et **R'₄**, identiques ou différents, représentent un atome d'hydrogène, un groupe hydroxyle, un groupe hydroxy(C₁-C₄)alkyle ou -C(O)OR' avec R' représentant un atome d'hydrogène, un groupe alkyle en C₁-C₄ ou alors un groupe -C(O)-O⁻ et dans ce cas un contre ion anionique An⁻ est absent, étant entendu qu'un seul de ces deux radicaux **R'₃** ou **R'₄** peut représenter un atome d'hydrogène ;
- **R₃** représente un atome d'hydrogène ou un groupement -C(O)OR" avec R" représentant un atome d'hydrogène, un groupe alkyle en C₁-C₄ ou alors R₃ représente un groupe -C(O)-O⁻ et dans ce cas un contre ion anionique An⁻ est absent ;
- **n** vaut 1 ou 2 ;
- **m** vaut 0 ou 1 ;
- **t** vaut 1 ou 2 ;
- **An⁻** représente un contre ion anionique ;
- **Y** représente : i) un atome d'hydrogène ; ii) un métal alcalin ; iii) un métal alcalino-terreux ; iv) un groupe ammonium : N⁺R^{α}R^{β}R^{γ}R^{δ}, An⁻ ou un groupe phosphonium : P⁺R^{α}R^{β}R^{γ}R^{δ}, An⁻ avec R^{α}, R^{β}, R^{γ} et R^{δ}, identiques ou différents, représentant un atome d'hydrogène ou un groupe (C₁-C₄)alkyle et An⁻ est tels que défini précédemment ; ou v) un groupe protecteur de fonction thiol ; étant entendu que :
   - la liaison entre le cycle pyridinium et la double liaison du groupe styryle est positionnée en position 2 ou 4 du pyridinium ;
   - lorsque n=1, m=1 alors Y = H ou groupe protecteur de la fonction thiol et lorsque n=2, alors m=0.

A titre d'exemple on peut citer les colorants fluorescents appartenant aux formules (I), (la) ou (Ib) suivants:

| | |
|---|---|
| 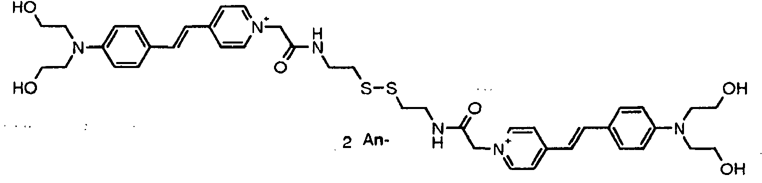 | 1 |
| 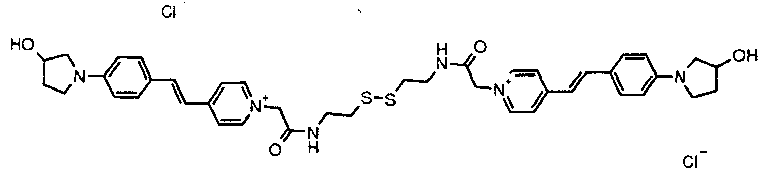 | 2 |
| 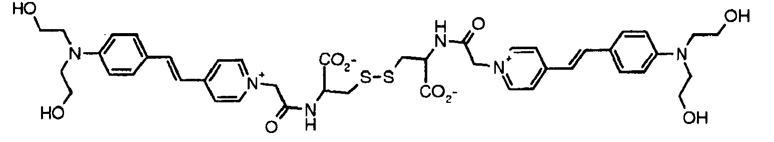 | 3 |
| 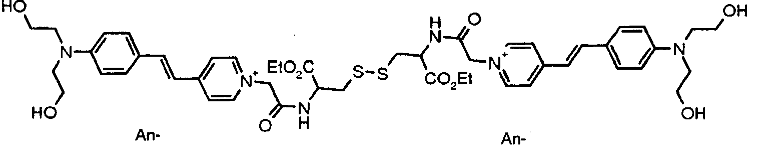 | 4 |
| 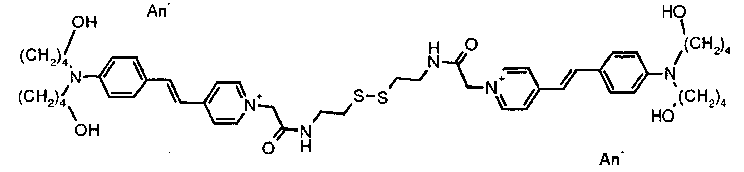 | 5 |
| 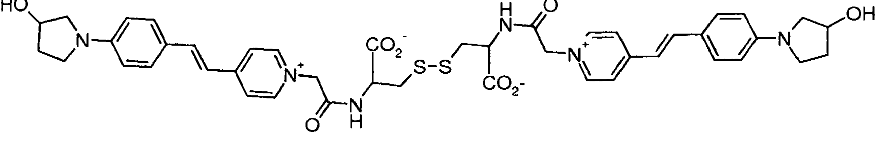 | 6 |
| 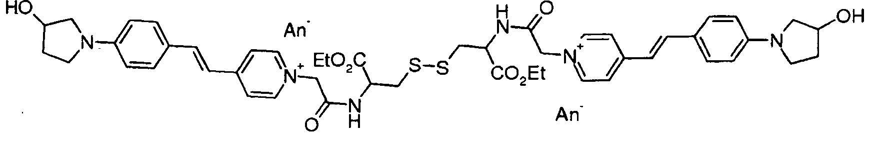 | 7 |
| 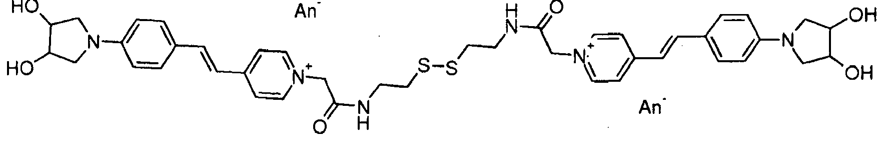 | 8 |
| 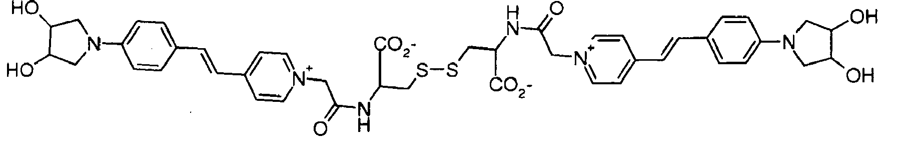 | 9 |
| 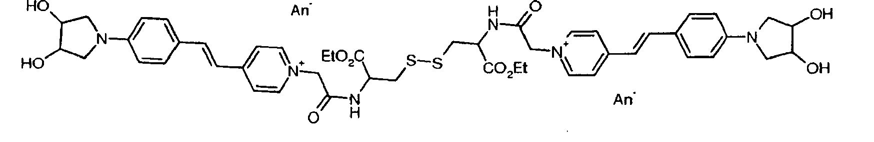 | 10 |
| 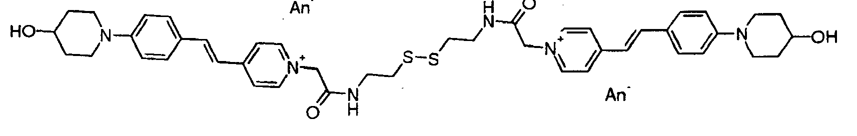 | 11 |
| 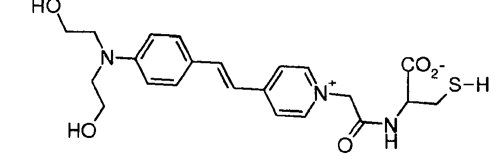 | 12 |
| 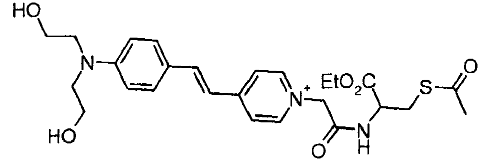 | 13 |
| 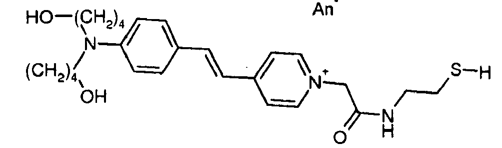 | 14 |
| 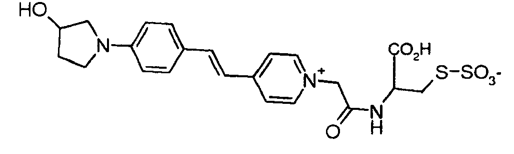 | 15 |
| 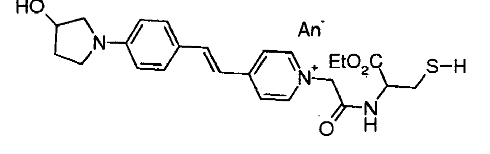 | 16 |
| 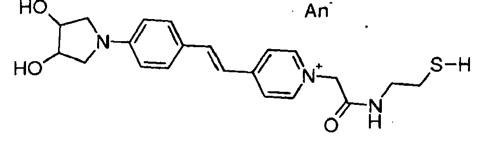 | 17 |
| 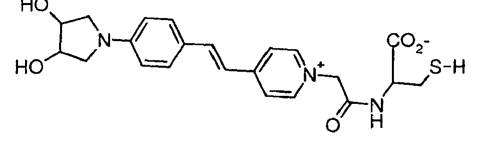 | 18 |
| 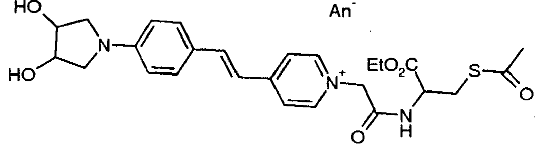 | 19 |
| 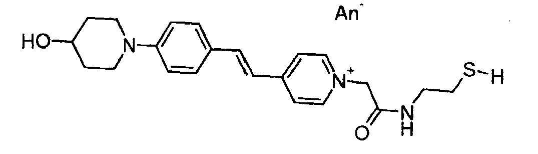 | 20 |
| 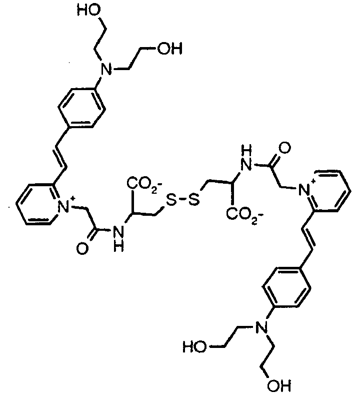 | 21 |
| 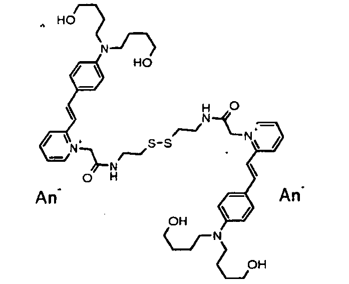 | 22 |
| 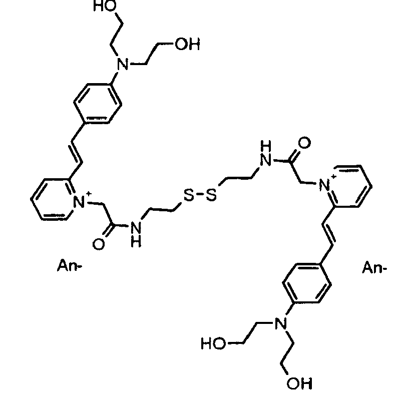 | 23 |
| 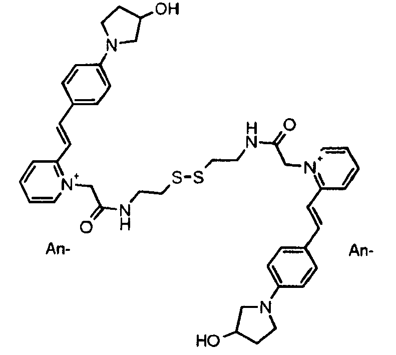 | 24 |
| 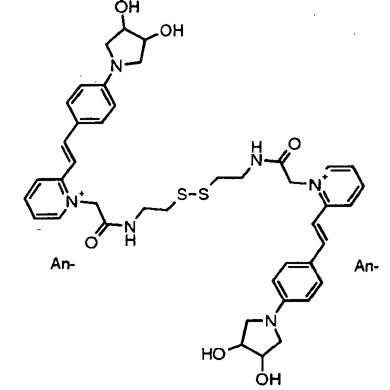 | 25 |
| 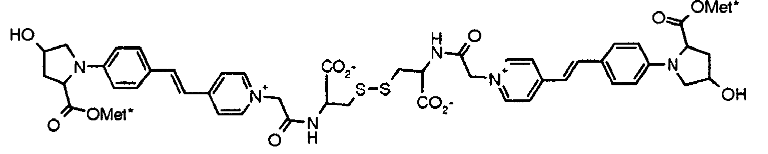 | 26 |
| 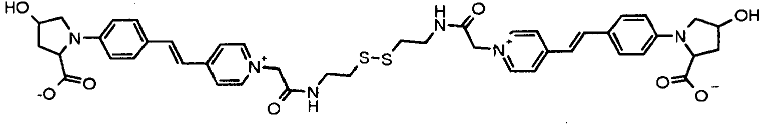 | 27 |
| 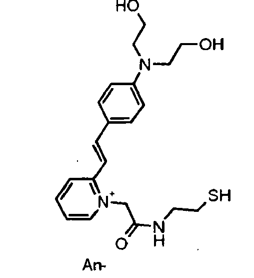 | 28 |
| 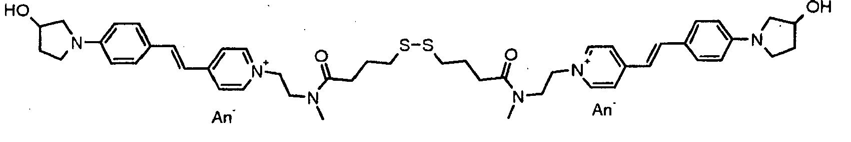 | 29 |
| 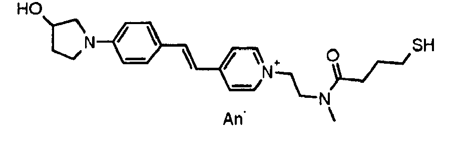 | 30 |
| 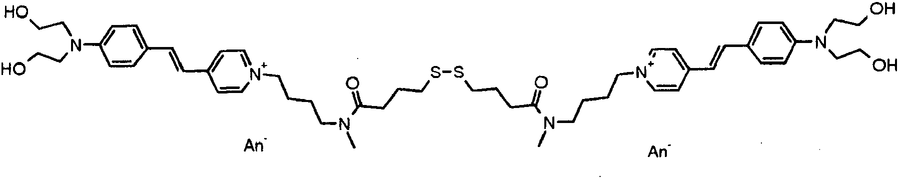 | 31 |
| 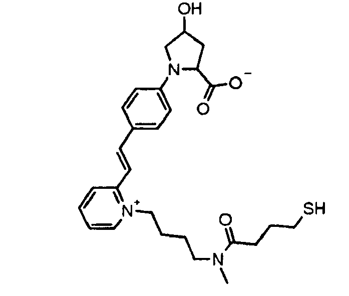 | 32 |
| 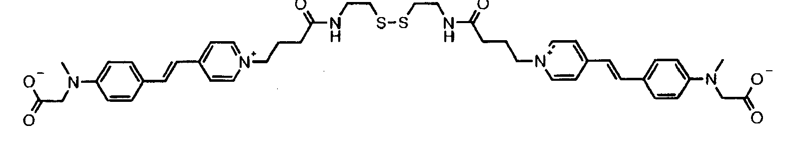 | 33 |
| 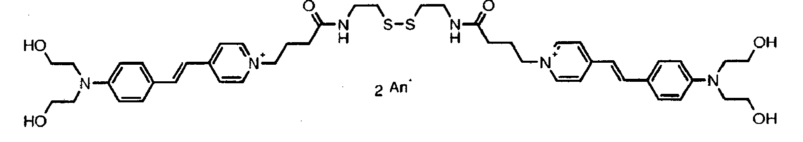 | 34 |
| 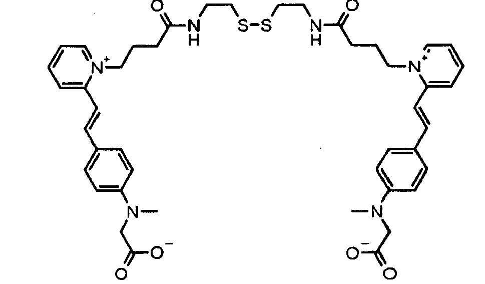 | 35 |
| 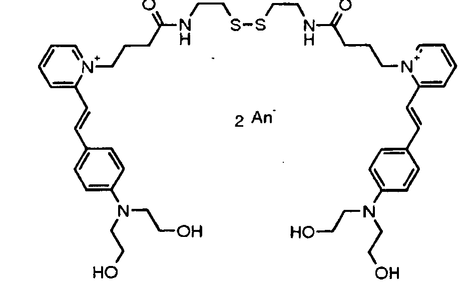 | 36 |

avec An⁻ représentant un contre-ion anionique.

Pour préparer les colorants fluorescents thiols/disulfure selon l'invention un peut citer les voies générales suivantes.

Les colorants thiols protégés de formule **(II')** peuvent être synthétisés en deux étapes. La première étape consistant à préparer le colorant thiol non protégé **(II")** selon les méthodes connues de l'homme de l'art comme par exemple « Thiols and organic Sulfides », « Thiocyanates and Isothiocyanates, organic », Ullmann's Encyclopedia, Wiley-VCH, Weinheim, 2005. Et la deuxième étape consistant à protéger la fonction thiol selon les méthodes classiques connues par l'homme du métier pour conduire aux colorants thiols protégés de formule **(II')**. A titre d'exemple pour protéger la fonction thiol -SH du colorant thiol on peut utiliser les méthodes des ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5.

Nous pouvons illustrer cette méthode par la méthode consistant i) à générer des colorants fluorescents thiols de formule **(II")** par réduction d'un colorant fluorescent à deux chromophores, hétérocyclique, portant une fonction disulfure -S-S- tels que **(I')** et ii) à protéger selon les méthodes classiques ladite fonction thiol de **(II")** avec le réactif **7** Y'R pour accéder aux colorants fluorescents thiols protégés de formule **(II')**. Le composé thiol **(II")** peut également être métallé avec un métal alcalin ou alcalino terreux Met* pour conduire au colorant fluorescent thiolate de formule **(II''')**. 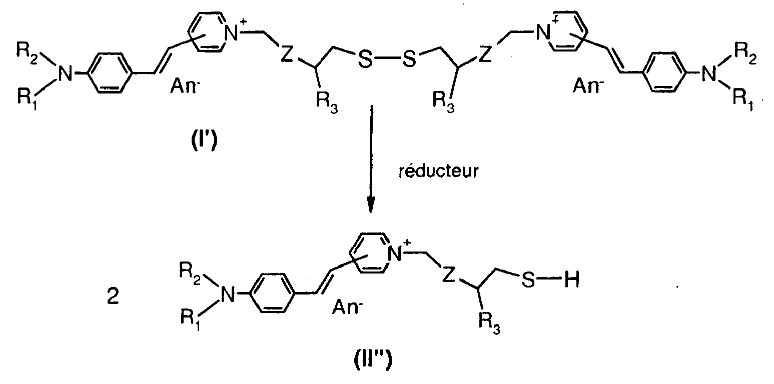 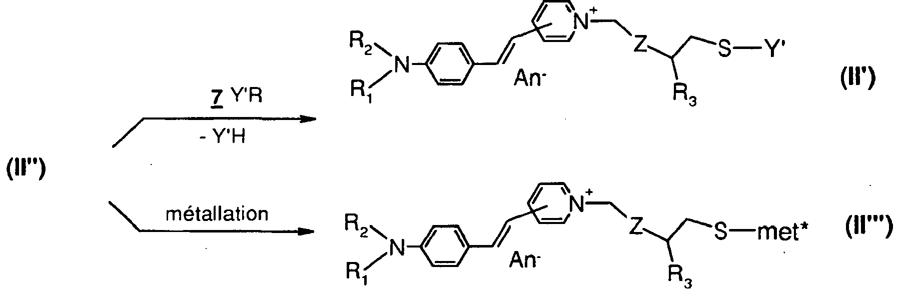 avec Y' représentant un groupe protecteur de fonction thiol ; Met* représentant un métal alcalin ou un métal alcalino térreux, particulièrement le sodium ou le potassium, étant entendu que lorsque le métal est un métal alcalino-terreux 2 chromophores à fonction thiolate-S⁻ peuvent être associés à 1 Métal²⁺. avec R₁, R₂, R₃, R₄, m, n, G et An⁻ sont tels que définis précédemment ; Y' représente un groupe protecteur de fonction thiol ; et R représente un groupe partant nucléofuge, comme par exemple mésylate, tosylate, triflate ou halogénure.

Selon une autre possibilité, on peut faire réagir un composé thiol protégé **(b)** par un groupe protecteur Y' tel que défini précédemment préparé selon une des procédures décrites dans les ouvrages cités précédemment, ledit composé thiol protégé comprenant au moins une fonction nucléophile avec une quantité suffisante, préférentiellement équimolaire, d'un "chromophore fluorescent réactif" ou d'un composé comprenant un tel "chromophore fluorescent réactif" **(a)**. En d'autres termes **(a)** comprend une fonction électrophile pour former une liaison covalente Σ comme cela peut être schématisé ci-dessous dans la préparation de colorants fluorescents de formule **(II'a)**, sous ensemble des colorants fluorescents (II'): 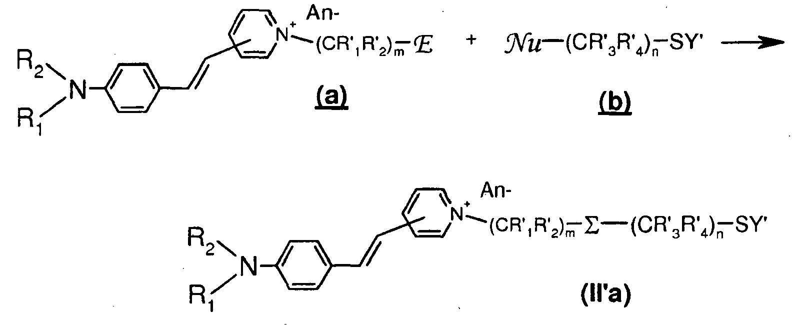 avec R₁, R₂, Y' et An⁻ sont tels que définis précédemment ; R'₁, R'₂, R'₃, R'₄ représentant des chaînes alkyles en C₁-C₄ éventuellement substituées par un groupement acide carboxylique, un sel d'acide carboxylique, une fonction ester, ou un groupement hydrogène, m et n entiers compris entre 1 et 4, *Nu* représentant un groupe nucléophile de type amine ; *E* représentant un groupe électrophile; et Σ le groupe de liaison générée après attaque du nucléophile sur l'électrophile.

A titre d'exemple, les liaisons Σ pouvant être générées sont répertoriées dans le tableau ci-dessous à partir de condensation d'électrophiles avec des nucléophiles :

| Electrophiles *E* | Nucléophiles *Nu* | Liaisons covalentes Σ |
|---|---|---|
| Esters activés* | Amines | Carboxamides |
| Azotures d'acyles | Amines | Carboxamides |
| Haloqénures d'acyles | Amines | Carboxamides |
| Cyanures d'acyles | Amines | Carboxamides |
| Anhydrides | Amines | Carboxamides |
| Acides carboxyliques | Amines | Carboxamides |

| | | |
|---|---|---|
| *les esters activées de formule générale -CO-Part avec Part représentant un groupe partant tel que oxysuccinimidyle, oxybenzotriazolyle, aryloxy éventuellement substitué ; | | |

On pourra également utiliser un réactif thiol (α) : Y'-SH comprenant un groupe Y' tel que défini précédemment dont la fonction nucléophile SH peut réagir sur l'atome de carbone en alpha de l'atome d'halogène porté par un chromophore fluorescent pour conduire au colorant fluorescent thiol protégé de formule **(II')**: 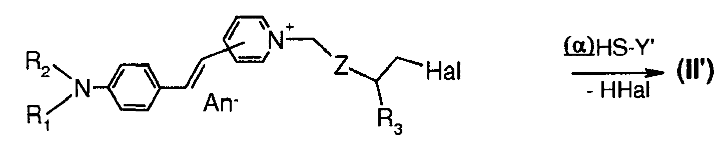 avec R₁, R₂, R₃, Z, Y', **(II')** et An⁻ sont tels que définis précédemment, et Hal représentant un atome d'halogène nucléofuge tel que le brome, l'iode ou le chlore.

Plus particulièrement on pourra substituer un groupe partant nucléofuge par un groupe dérivé de la thiourée (S=C(NRR)NRR) ou la thiourée pour générer les isothiouroniums. Par exemple si le groupe thiourée est une thioimidazolium **(β)**, le schéma réactionnel est le suivant : 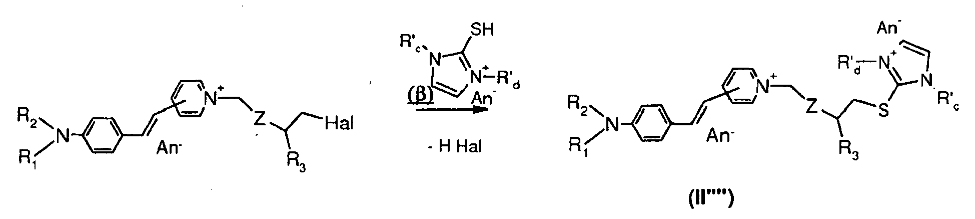 avec R₁, R₂, R₃, Z, Hal et An sont tels que définis précédemment, R'_{c} et R'_{d} représentant des groupements alkyles en C₁-C_{4;} **(II"")** représentant un colorant sous ensemble de la formule **(II')**

Une autre variante peut permettre d'accéder à certains composés **(II')** à partir de la thiourée cyclique de type imidazoline **(*b*')**, suivi de l'alkylation de ladite imidazoline à l'aide de R'_{d}-Gp avec Gp groupe partant tel que chlorure, bromure, tosylate, mésylate : 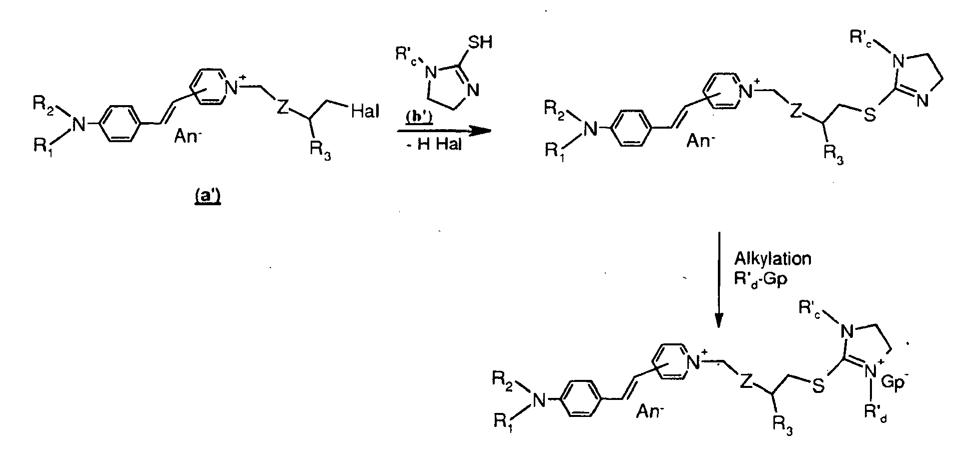 avec R₁, R₂, R₃, Z, R'_{c}, R'_{d}, **(II')**, Hal et An-, sont tels que définis précédemment.

Une variante est d'utiliser à la place de l'halogénure comprenant le chromophore fluorescent **(a')** un chromophore comprenant un autre type de nucléofuge tel que le tosylate, mésylate.

Conformément à une autre possibilité, certains colorants fluorescents thiols protégés **(II'a)** peuvent être obtenus en faisant réagir un composé thiol protégé, avec un composé portant une fonction acide carboxylique activée selon les méthodes classiques (par exemple réaction avec un carbodiimide ou avec le chlorure de thionyle). Le produit résultant **(d)** est ensuite mis à réagir avec un chromophore fluorescent porteur d'une fonction nucléophile **(c)**, de type amine primaire ou secondaire. 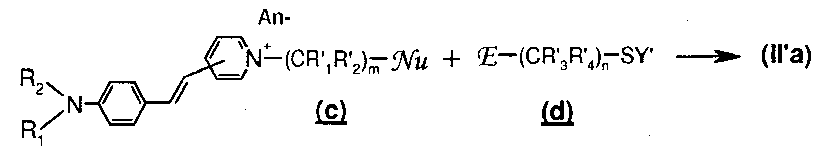 avec R₁, R₂, R'_{c}, R'_{d}, An-, R'₁, R'₂, R'₃, R'₄, Y', m, n, *E*, *Nu* et **(II'a)** tels que définis précédemment.

Une autre variante est d'utiliser un dérivé de thiolactone tel que représenté par le schéma ci-dessous :

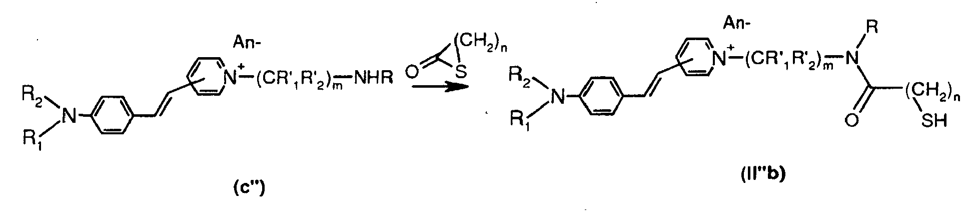

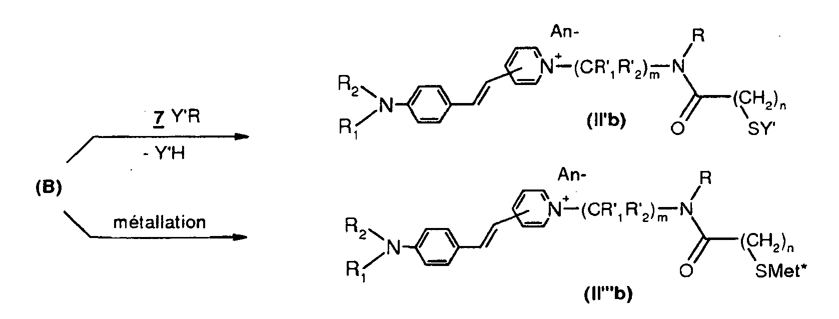

Avec R₁, R₂, R'₁, R'₂ Y', Met*, m, n An⁻, tels que définis précédemment. Le dérivé thiolactone est préférentiellement choisi avec n=3.

Une variante de synthèse est de combiner à la première voie la voie précédente ie à partir de deux équivalents du réactif nucléophile **(c)** avec un réactif diéléctrophile disulfure **(i)** il est possible de générer après condensation le produit dichromophorique disulfure **(I)**, ce dernier pouvant subir une réduction pour former le colorant thiol fluorescent hétérocyclique (II"a) qui a son tour peut soit être protégé pour former le colorant fluorescent thiol protégé **(II'a)** ou soit être métallé par un métal alcalin pour conduire au colorant fluorescent thiol hétérocyclique métallé **(II'''a)** :

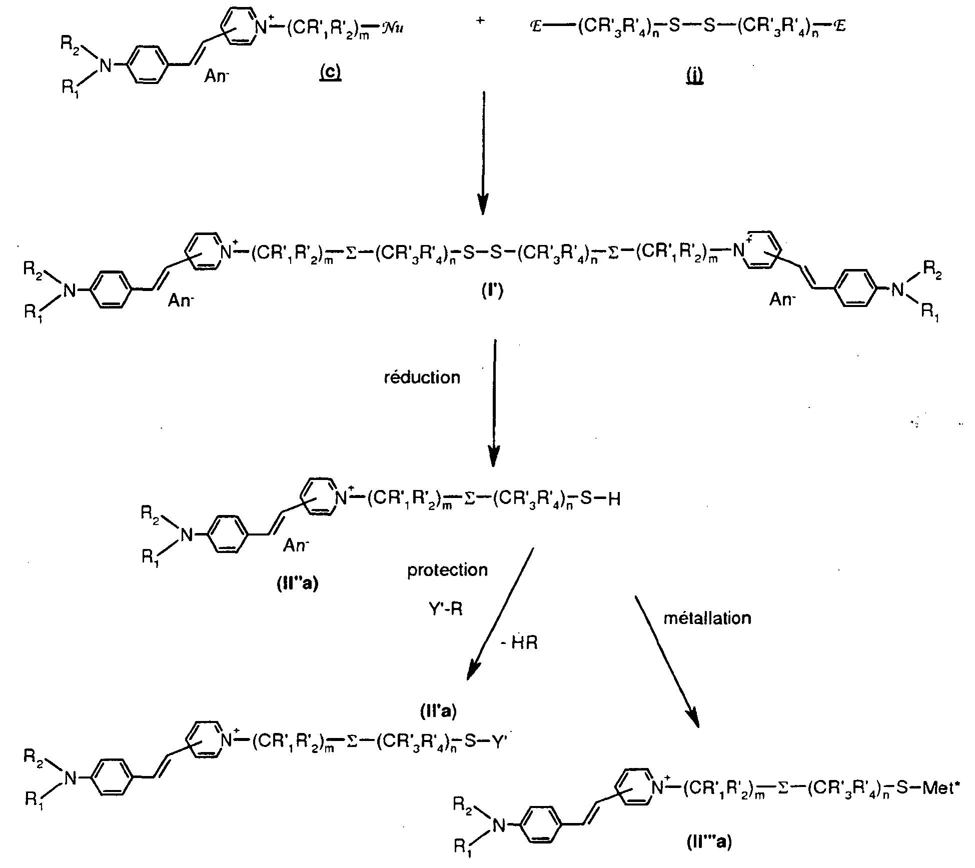

Conformément à une autre possibilité, les colorants fluorescents thiols protégés de formule **(II')** peuvent être obtenus par réaction d'un composé **d'** comprenant un groupe thiol protégé par un groupe Y', et un groupement partant Gp, tel q'un groupement halogènes (chlore, brome) ou un groupement hydroxy activé préalablement en groupe partant nucléofuge comme par exemple mésylate, tosylate, triflate ou halogénure avec un chromophore styrylpyridine **(c')**. 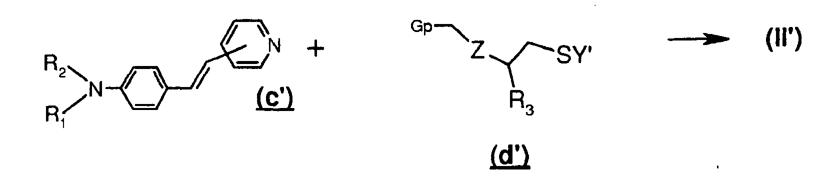 Avec R₁, R₂, R₃, Z, Y', , **(II')** et sont tels que définis précédemment.

Conformément à une autre possibilité, les colorants fluorescents thiols de formule **(I)** selon l'invention peuvent être obtenus par réaction d'un composé comprenant un groupe thiol protégé par un groupement Y tel que défini précédemment et un groupe électrophile **(f)** avec un composé pyridinium comprenant un groupe nucléophile. A titre d'exemple, on pourra condenser un aldéhyde ou un thioaldéhyde lorsque G' représente un atome d'oxygène ou un soufre avec un « méthylène activé » tel que l'alkylpyridinium **(e)** pour générer une liaison éthylène >C=C<. Cette réaction est communément appelée condensation de « Knoevenagel ». Par « méthylènes activés » on sous entend ceux qui comporte en position 2 ou 4 du groupe pyridinium un groupe méthylène CH₃-: 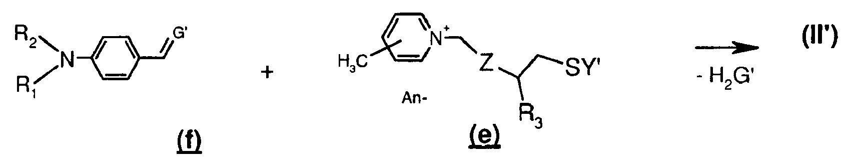 avec R₁, R₂, R₃, Z, Y' An⁻ tels que définis précédemment et G' représentant un atome d'oxygène ou de soufre.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupes protecteurs classiques. Les colorants fluorescents thiols sont métallés en utilisant également les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Les réactifs de départs sont commerciaux ou accessibles par les méthodes classiques connues par l'homme du métier. A titre d'exemple il est possible de synthétiser le réactif **(I')** à partir de 2 équivalents de dérivé pyridinique **1** et un équivalent de réactif disulfure comprenant deux groupes partants Gp, pour conduire au sel de dipyridinium disulfure **3** qui peut se condenser à son tour avec deux équivalents de composé aryle à groupe aldéhyde/thioaldéhyde **4** pour conduire à **5.** 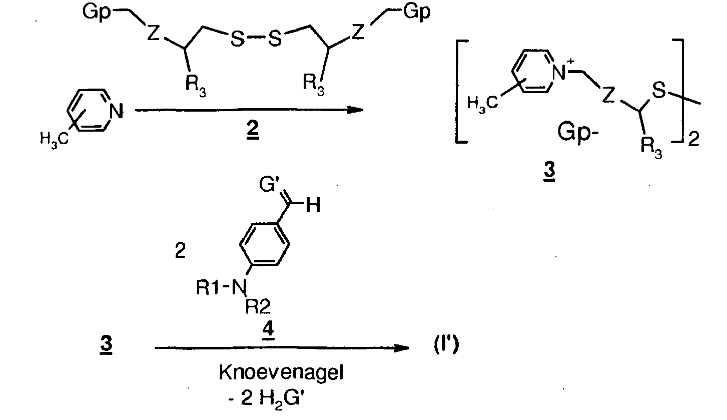 avec Gp représentant un groupe partant nucléofuge, comme par exemple mésylate, tosylate, triflate ou halogénure. Les contre-ions Gp⁻ des composés **(I')**, ci-dessus peuvent être remplacés par des contre-ions An⁻ d'autres natures à partir de méthodes connues par l'homme du métier notamment par résine échangeuse d'ion.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupes protecteurs classiques. Les colorants fluorescents thiols sont métallés en utilisant également les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Un autre objet de l'invention concerne une composition tinctoriale pour colorer les matières kératiniques qui contient au moins un colorant fluorescent thiol/disulfure de formule **(I)**. Outre la présence d'au moins un colorant fluorescent de formule (I), la composition de l'invention peut également contenir un agent réducteur.

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catéchol-borane.

La composition tinctoriale utile dans l'invention contient en général une quantité de colorant fluorescent de formule **(I)** comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition.

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10% en poids du poids total de la composition tinctoriale, de préférence entre 0,005 et 6%.

La ou les bases d'oxydation présentes dans la composition tinctoriale sont en général présentes chacune en quantité comprise entre 0,001 à 10% en poids du poids total de la composition tinctoriale, de préférence entre 0,005 et 6% en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants autres que l'eau lorsqu'ils sont présents sont, de préférence présents dans des proportions de préférence comprises entre 1 et 40% en poids environ par rapport au poids total de la composition tinctoriale, et encore plus préférentiellement entre 5 et 30% en poids environ. Les solvants y compris l'eau sont présents, de préférence présents dans des proportions de préférence comprises entre 1 et 99% en poids environ par rapport au poids total de la composition tinctoriale, et encore plus préférentiellement entre 5 et 95% en poids environ.

Selon une variante, la composition de l'invention contient un agent réducteur capable de réduire les liaisons disulfures de la kératine et/ou disulfure des colorants fluorescents de formule (I). Cet agent réducteur est tel que défini précédemment.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

Les adjuvants ci dessus sont en général présents en quantité comprise pour chacun d'eux entre 0,01 et 20% en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (γ) suivante : 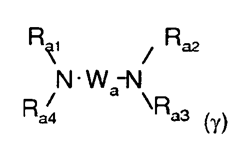 dans laquelle Wₐ est un reste propylène éventuellement substitué par un groupe hydroxy ou un radical alkyle en C₁-C₄ ; Rₐ₁, Rₐ₂, Rₐ₃ et Rₐ₄, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Un autre objet de l'invention est un procédé de coloration de matière kératinique consistant à appliquer sur les dites matières une composition comprenant au moins un colorant de formule (I). Selon un mode de réalisation particulier dans le procédé de l'invention un agent réducteur peut également être appliqué en pré-traitement avant l'application de la composition contenant au moins un colorant fluorescent de formule (I).

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catécholborane.

Ce prétraitement peut être de courte durée, notamment de 1 seconde à 30 minutes, de préférence de 1 minute à 15 minutes avec un agent réducteur tel que cité précédemment.

Selon un autre procédé, la composition comprenant au moins un colorant fluorescent de formule (I) et contient également au moins un agent réducteur tel que défini précédemment. Cette composition est alors appliquée aux cheveux.

Lorsque le colorant fluorescent thiol de formule (I) avec n=m=1 comprend un groupe Y protecteur de la fonction thiol, le procédé de l'invention peut être précédé d'une étape de déprotection visant à restituer *in-situ* la fonction SH.

A titre d'exemple il est possible de déprotéger la fonction S-Y avec Y groupe protecteur en ajustant le pH comme suit :

| Y : groupe protecteur | déprotection |
|---|---|
| | |
| alkylcarbonyle, | pH>9 |
| arylcarbonyle, | pH>9 |
| alkoxycarbonyle, | pH>9 |
| aryloxycarbonyle, | pH>9 |
| arylalkoxycarbonyle | pH>9 |
| (di)(alkyl)aminocarbonyle, | pH>9 |
| (alkyl)arylaminocarbonyle | pH>9 |
| aryle éventuellement substitué tel que le phényle, | pH>9 |
| hétéroaryle monocyclique à 5, 6 ou 7 chaînons tel que l'oxazolium ; | pH>9 |
| hétéroaryle bicyclique à 8 à 11 chaînons tels que benzoimidazolium, ou benzoxazolium | pH>9 |

L'étape de déprotection peut également être réalisée au cours d'une étape de prétraitement des cheveux comme par exemple, le prétraitement réducteur des cheveux.

Selon une variante, l'agent réducteur est ajouté à la composition tinctoriale contenant au moins un colorant fluorescent de formule (I) au moment de l'emploi.

Selon un autre procédé, la composition comprenant au moins un colorant fluorescent de formule (I) contient également au moins un agent réducteur tel que défini précédemment. Cette composition est alors appliquée aux cheveux.

Selon une autre variante, l'agent réducteur est appliqué en post-traitement, après l'application de la composition contenant au moins un colorant fluorescent de formule (I). La durée du post traitement avec l'agent réducteur peut être courte, par exemple de 1 seconde à 30 minutes de préférence de 1 minute à 15 minutes, avec un agent réducteur tel que décrit précédemment. Selon un mode de réalisation particulier l'agent réducteur est un agent de type thiol ou borohydrure tel que décrit précédemment.

Un mode de réalisation particulier de l'invention concerne un procédé dans lequel le colorant fluorescent de formule (I) peut être appliqué directement aux cheveux sans réducteurs, exempt de pré ou post-traitement réducteurs.

Un traitement avec un agent oxydant peut éventuellement être associé. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons tels que les uricases et les oxygénases à 4 électrons comme les laccases. L'utilisation du peroxyde d'hydrogène est particulièrement préférée.

Cet agent oxydant peut être appliqué sur les fibres avant ou après l'application de la composition contenant au moins un colorant fluorescent de formule (I). Un mode particulier de l'invention concerne le procédé de l'invention comprenant une étape supplémentaire qui consiste à appliquer sur les fibres kératiniques un agent oxydant.

L'application de la composition tinctoriale selon l'invention est généralement effectuée à température ambiante. Elle peut cependant être réalisée à des températures variant de 20 à 180 °C.

L'invention a aussi pour objet un dispositif à plusieurs compartiments ou "kit" de teinture dans lequel un premier compartiment renferme une composition tinctoriale comprenant au moins un colorant fluorescent de formule (I) et un deuxième compartiment renferme un agent réducteur capable de réduire les fonctions disulfures des matières kératiniques et/ou du colorant fluorescent disulfure de formule (I), lorsque n vaut 2 et m vaut 0.

L'un de ces compartiments peut en outre contenir un ou plusieurs autres colorants de type colorant direct ou colorant d'oxydation.

Elle concerne aussi un dispositif à plusieurs compartiments dans lequel un premier compartiment contient une composition tinctoriale comprenant au moins un colorant fluorescent de formule (I) ; un deuxième compartiment contient un agent réducteur capable de réduire la liaison disulfure des matières kératiniques et/ou du colorant fluorescent disulfure de formule (I) lorsque n vaut 2 et m vaut 0 ; un troisième compartiment contient un agent oxydant.

Alternativement, le dispositif de teinture contient un premier compartiment renfermant une composition tinctoriale qui comprend au moins un colorant fluorescent thiol protégé de formule (I) lorsque n=m=1 et un deuxième compartiment renfermant un agent capable de déprotéger le thiol protégé pour libérer le thiol.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif. Les colorants fluorescents thiols des exemples ci-après ont été entièrement caractérisés par les méthodes spectroscopiques et spectrométriques classiques.

### EXEMPLES

### EXEMPLES DE SYNTHESE

### Exemple 1 : Synthèse du dichlorure de 1,1'-{disulfanediylbis[éthane-2,1-diylimino(2-oxoéthane-2,1-diyl)]}bis[4-(-2-{4-[bis(2-hydroxyéthyl)amino]phényl}vinyl)pyridinium]

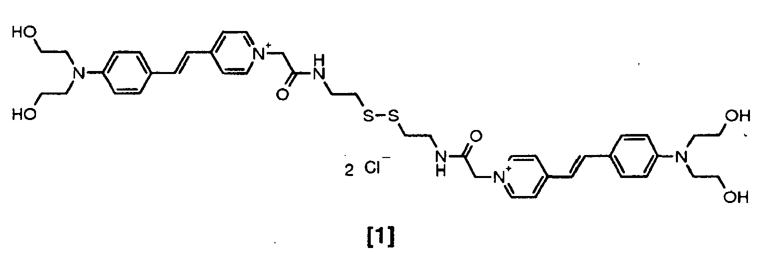

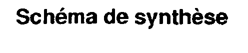

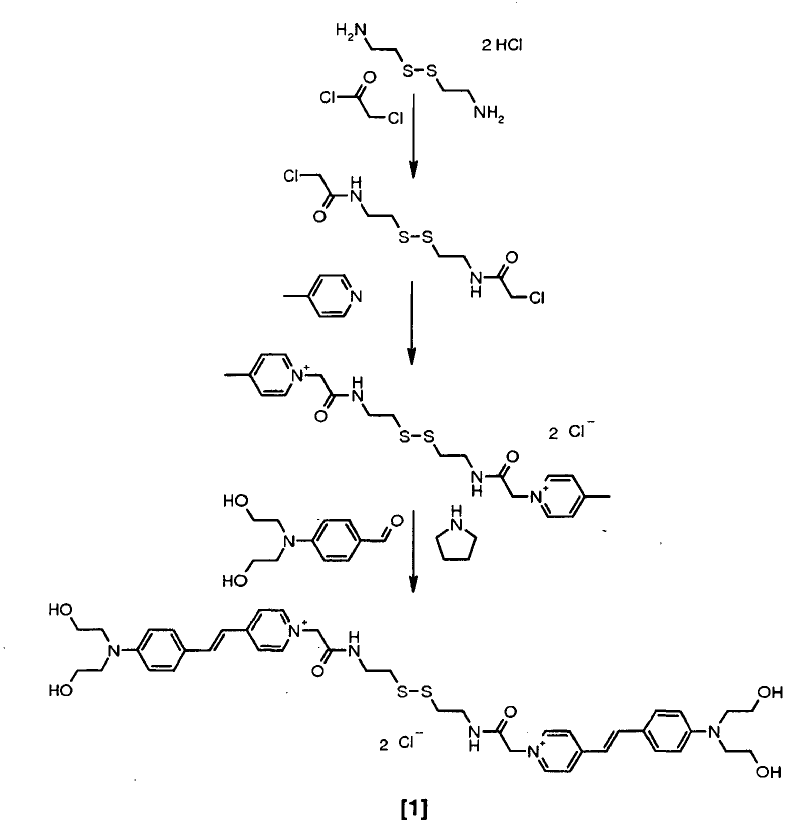

### Etape 1 : Synthèse du N,N'-(disulfanediyldiéthane-2,1-diyl)bis(2-chloroacétamide)

40.3 g de dichlorhydrate de cystamine sont dissous dans 100 mL d'eau, 32 mL de soude à 35% sont ajoutés (pH 9.7) et la température est abaissée à 5°. 33.5 mL de chlorure de chloracétyle sont introduits goutte à goutte, en maintenant la température inférieure à 10°C et en maintenant le pH entre 7.9 et 9.3 par addition de soude. Le milieu est maintenu agité à température ambiante pendant 2 H. Le précipité est filtré, lavé par 5 x 150 mL d'eau puis séché sous vide en présence de P₂O₅. 35.3 g de poudre blanche sont recueillis. les analyses indiquent que le produit est conforme.

### Etape 2 : Synthèse du dichlorure de 1,1'-{disulfanediylbis[éthane-2,1-diylimino(2-oxoéthane-2,1-diyl)]}bis(4-méthylpyridinium)

6.1 g N,N'-(disulfanediyldiéthane-2,1-diyl)bis(2-chloroacétamide) et 4.5 g de 4-picoline sont dissous dans 50 mL de NMP et portés à 80°C pendant 19 h. Après refroidissement du mélange, par précipitations successives dans l'acétone et séchage sous vide, 9.2g de sels sont recueillis. Les analyses montrent que le produit est conforme. RMN ¹H (400 MHz, D₂O) : 2.61 (s, 6 H), 2.82 (t, 4 H), 3.56 (t, 4 H), 5.31 (s, 4 H), 7.85 (d, 4 H), 8.51 (d, 4 H).

### Etape 3 : Synthèse du dichlorure de 1,1'-{disulfanediylbis[éthane-2,1-diylimino(2-oxoéthane-2,1-diyl)]}bis[4-(2-{4-[bis(2-hydroxyéthyl)amino]phényl}vinyl)pyridinium] [1]

837 mg de 4-[bis-(2-hydroxy-éthyl)-amino]-benzaldéhyde, 328 µL de pyrrolidine, 232 µL d'acide acétique et 490 mg de dichlorure de 1,1'-{disulfanediylbis[éthane-2,1-diylimino(2-oxoéthane-2,1-diyl)]}bis(4-méthylpyridinium) sont mis en solution dans 10 mL d'isopropanol et maintenus agités à température ambiante pendant 3 h 30 min. Le mélange est versé sur 50 mL de solution de dichlorométhane / acétone 1 :1. Une huile décante, elle est séchée sous vide. 442 mg de poudre noire sont recueillis. Les analyses montrent que le produit est conforme.

### Exemple 2 : dichlorure de 1,1'-{disulfanediylbis[éthane-2,1-diylimino(2-oxoéthane-2,1-diyl)]}bis(4-{2-[4-(3-hydroxypyrrolidin-1-yl)phenyl]vinyl}pyridinium)

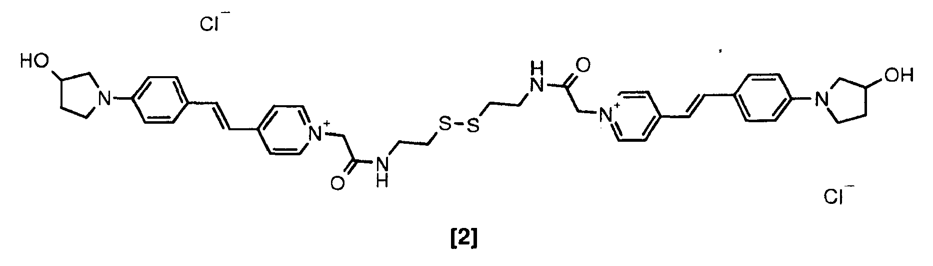

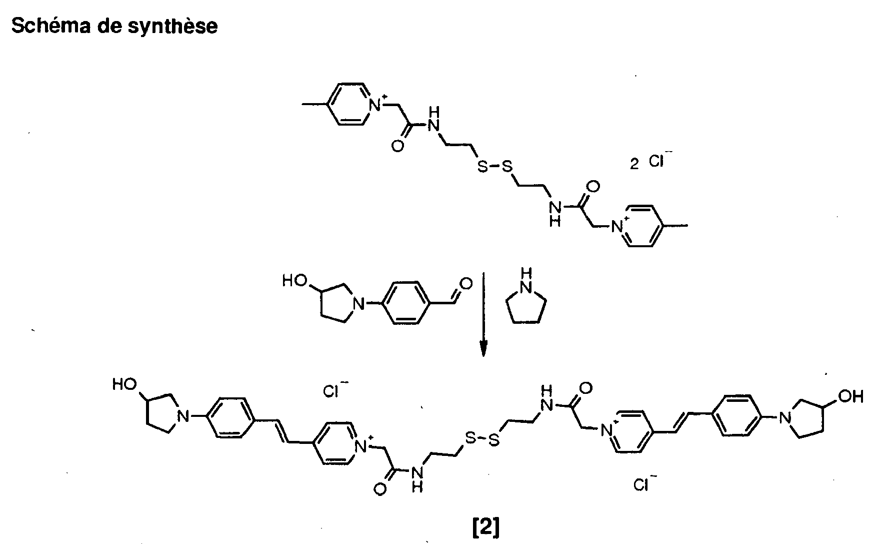

19.88 g de 4-(3-hydroxypyrrolidin-1-yl)benzaldéhyde, 7.17g de pyrrolidine, 6.05g d'acide acétique et 24.8g de dichlorure de 1,1'-{disulfanediylbis[éthane-2,1-diylimino(2-oxoéthane-2,1-diyl)]}bis(4-méthylpyridinium) sont mis en solution dans 200 mL d'éthanol et maintenus agités à température ambiante pendant 48h. Le mélange est filtré, Ilavé avec 3 fois 100mL d'isopropanol. 43.19g de poudre noire sont recueillis puis recristallisés dans un mélange de 200 mL d'éthanol, 50 mL de méthanol et 25 mL d'eau. Après filtration et séchage sous vide, 35,8g de poudre rouge brique sont obtenus. Les analyses montrent que le produit est conforme.

### Exemple 3 : synthèse du diacétate de 2,2'-{disulfanediylbis[ethane-2,1-diylimino(4-oxobutane-4,1-diyl)pyridinium-1,4-diyléthène-2,1-diylbenzène-4,1-diyl(méthylimino)]}

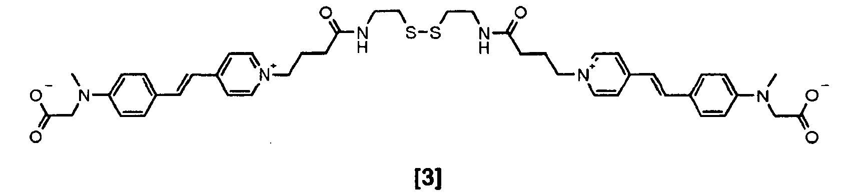

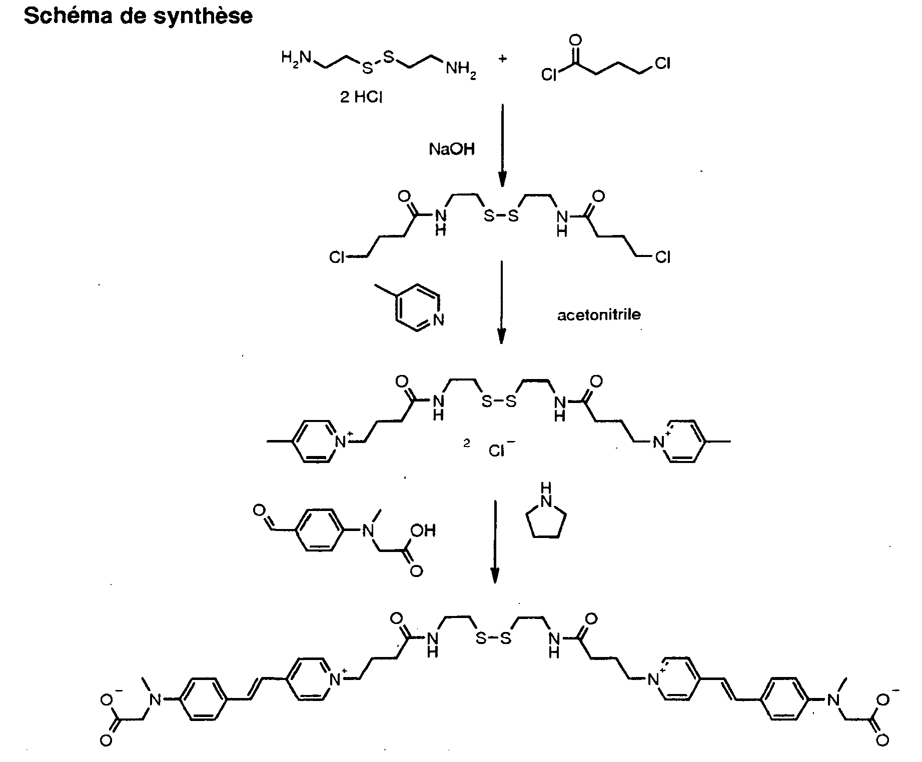

### Etape 1 : synthèse du N,N'-(disulfanediyldiéthane-2,1-diyl)bis(4-chlorobutanamide)

60 g d'hydrochlorure de cystamine sont solubilisés dans 500 mL et refroidit à 5 °C. Le pH de 10 est atteint par addition de NaOH (30% aq.). A une solution de chlorure 4-chlorobutanoyl (105 g) dans du THF anhydre (500mL), est ajouté goutte à goutte du aq. NaOH (30%) de façon à maintenir le pH autour de 7. Après que la réaction soit stabilisée à un pH de 7, le mélange est laissé pendant 3 jours à température ambiante. La phase aqueuse est extraite par 3 x 500 m de dichlorométhane associé à la phase de THF puis séchée sur sulfate de sodium Na₂SO₄. Après filtration et séchage, 41 g de poudre blanche est recueillie. Les analyses sont en accord avec le produit attendu.

### Etape 2 : Synthèse du dichlorure de 1,1'-{disulfanediylbis[éthane-2,1-diylimino(4-oxobutane-4,1-diyl)]}bis(4-méthyl-pyridinium)

30 g de N,N'-(disulfanediyldiéthane-2,1-diyl)bis(4-chlorobutanamide) sont dispercés dans 100 mL d'acétonitrile, 20.2 ml de 4-pioline sont ajoutés dans le mélange réactionnel sous agitation à 80 °C pour 2 jours. Après refroidissement à température ambiante, le solvant est évaporé et l'huile résultante est lavée plusieurs fois à l'acétate d'éthyle. 44.2g de solide marron clair sont obtenus. Les analyses sont en accord avec la structure du compose attendu.

### Etape 3 : Synthèse de diacétate de 2,2'-{disulfanediylbis[éthane-2,1-diylimino(4-oxobutane-4,1-diyl)pyridinium-1,4-diyléthène-2,1-diylbenzène-4,1-diyl(méthylimino)]}

16.7 g d'acide acétique [(4-formylphényl)(méthyl)amino]acétique et 6.3 g de pyrrolidine sont mélanges dans 50 mL d'isopropanol à 80 °C. Après 15 min. d'agitation, 1.46 g de pyrrolidine sont ajoutés suivi de l'ajout d'une solution de 22.5 g de dichlorure de 1,1'-{disulfanediylbis[éthane-2,1-diylimino(4-oxobutane-4,1-diyl)]}bis(4-méthylpyridinium) dans 100 mL d'isopropanol. Le mélange réactionnel est chauffé pendant 2 h. puis le mélange est refroidit à température ambiante, puis il est versé dans 1.4 L d'acétone. Un précipité se forme alors. Ce dernier est filtré sous argon, lavé 3 fois avec 170 mL d'acétone puis séché. Le solide résultant est dispersé dans 400 mL d'isopropanol et 400 mL d'eau puis 1.2L d'acétone sont ajoutés. L'huile résiduelle est dissoute dans 50 mL de méthanol et 750 mL d'acétone sont ajoutés. Une huile marron clair collante est triturée à l'aide de 500 mL d'acétone, filtrée et séchée. 20.5 g de poudre noire sont recueillis. Les analyses sont en accord avec la structure du produit attendu. NMR ¹H CD₃OD ppm : 2.33 (m, 4H), 2.48 (t, 4H), 2.98 (t, 4H), 3.31 (s, 6H), 3.59 (t, 4H), 4.54 (t, 4H), 6.83 (d, 4H), 6.99 (d, 2H), 7.59 (d, 4H) 7.75 (d, 2H), 7.86 ( d, 4H), 8.56 (d, 4H).

### Exemple 4 : synthèse du dichlorure de 1,1'-{disulfanediylbis[éthane-2,1-diylimino(4-oxobutane-4,1-diyl)]}bis{4-[2-{4-[bis-(2-hydroxyéthyl)amino]phényl}éthényl]pyridinium} dichloride

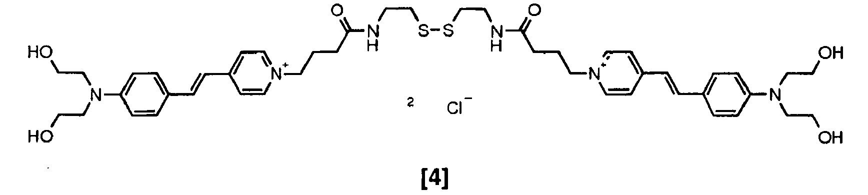

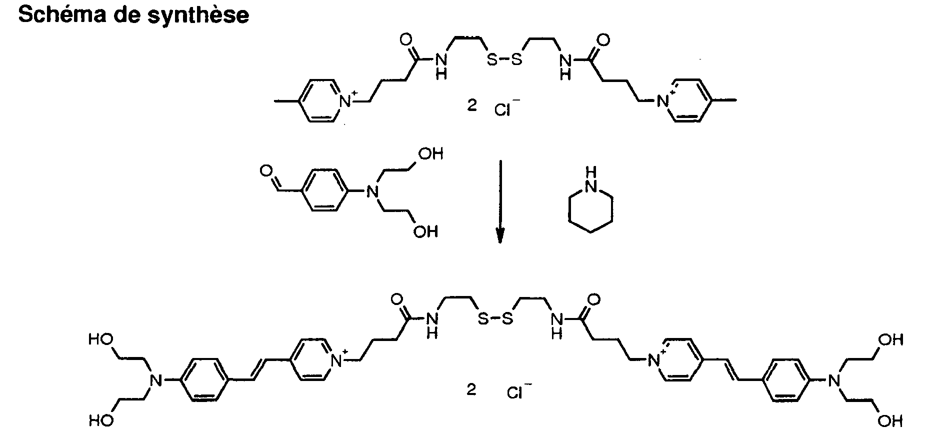

20g de 1,1'-{disulfanediylbis[éthane-2,1-diylimino(4-oxobutane-4,1-diyl)]}bis(4-méthyl-pyridinium)dichloride, 1.8 mL de pipéridine et 16 g de 4-[bis-(2-hydroxy-éthyl)-amino]-benzaldéhyde sont mélangés ensemble dans 60 mL d'isopropanol à 70 °C pendant 18 h sous agitation. Après refroidissement à température ambiante le surnageant est retiré, l'huile résultante est lavée 3 fois dans de l'isopropanol chaud (100 mL, 2h à 80 °C). L'huile est diluée dans 500 mL d'eau, glacée et lyophilisée. 24 g de poudre rouge "floconneuse" sont recueillis. Les analyses sont en accord avec la structure du produit attendu. NMR ¹H CD₃OD ppm : 2.25 (m, 4H), 2.36 (t, 4H), 2.81 (t, 4H), 3.45 (t, 4H), 3.64 (t, 8H), 3.76 (t, 8H), 4.46 (t, 4H), 6.83 (d, 4H), 7.07 (d, 2H), 7.59 (d, 4H), 7.82 (d, 2H), 7.96 (d, 4H), 8.57 (d, 4H), LC/MS gradient ACONH₄ 20mM->CH₃CN 10min ESI+ m/z=429

### Exemple 5 : synthèse du diacetate de 2,2'-{disulfanediylbis[éthane-2,1-diylimino(4-oxobutane-4,1-diyl)pyridinium-1,2-diyléthène-2,1-diylbenzène-4,1-diyl(méthylimino)]}

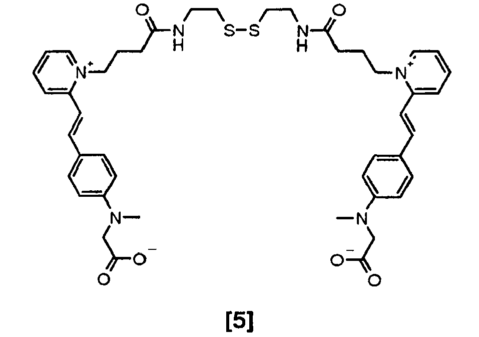

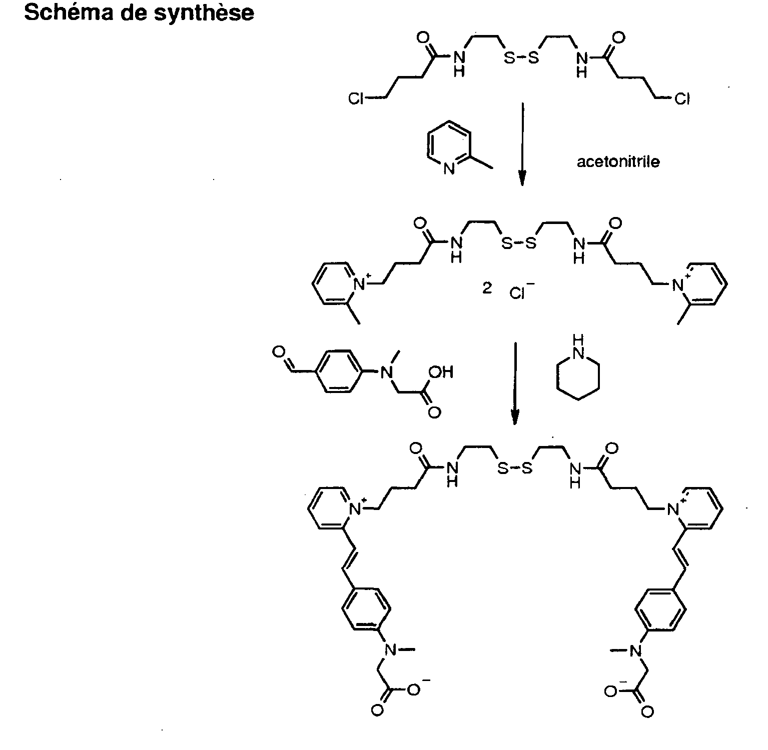

### Etape 1 : Synthesis dichlorure de 1,1'-{disulfanediylbis[éthane-2,1-diylimino(4-oxobutane-4,1-diyl)]}bis(2-méthyl-pyridinium)

1.13 g de *N*,*N*'-(disulfanediyldiéthane-2,1-diyl)bis(4-chlorobutanamide) sont dispersés dans 0.5 mL de N-méthylpyrrolidinone et 2.5 mL d'acétonitrile, puis chauffés à 50 °C. 0.71 mL de picoline sont alors ajoutés et le mélange réactionnel est agité pendant 5 jours à 50 °C. Quelques milligrammes de carbonate de césium et 0.35 mL de 2-picoline sont ajoutés. Après 1 jour à 65 °C, le mélange réactionnel est versé dans 50 mL d'acétonitrile. La gomme résultante est lavée plusieurs fois à l'acétate d'éthyle, solubilisée dans de l'éthanol, filtrée et séchée sous vide. 0.95 g de solide beige sont recueillis. Les analyses sont en accord avec la structure du compose attendu. LC/MS gradient ACONH₄ 20mM->CH₃CN 10min ESI+ m/z= 238 (dication)

### Etape 2 : Synthèse du diacétate de 2,2'-{disulfanediylbis[éthane-2,1-diylimino(4-oxobutane-4,1-diyl)pyridinium-1,2-diyléthène-2,1-diylbenzène-4,1-diyl(méthylimino)]}

0.35g d'acide [(4-formylphényl)(méthyl)amino]acétique, 0.47 g de dichlorure de 1,1'-{disulfanediylbis-[éthane-2,1-diylimino(4-oxobutane-4,1-diyl)]}bis(4-méthylpyridinium) et 42µL de pipéridine sont mélangés dans 3 mL de méthanol à 70 °C pendant 2 h. Le mélange réactionnel est verse dans 25 mL d'acétate d'éthyle puis le précipité est filtré et séché. Un poudre noire est obtenue. Les analyses sont en accord avec la structure du produit attendu. LC/MS gradient ACONH₄ 20mM->CH₃CN 10min ESI+ m/z=413

### Exemple 6 : synthèse de dichlorure de 1,1'-{disulfanediylbis[éthane-2,1-diylimino(4-oxobutane-4,1-diyl)]}bis{2-[-2-{4-[bis(2-hydroxyéthyl)amino]phényl}éthényl]pyridinium}

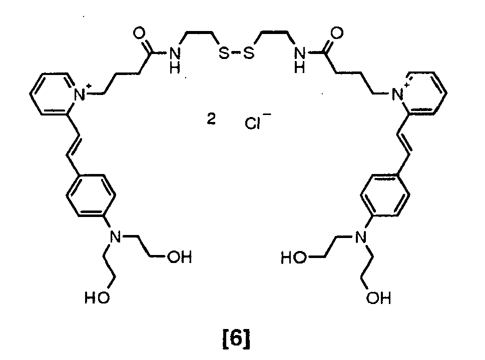

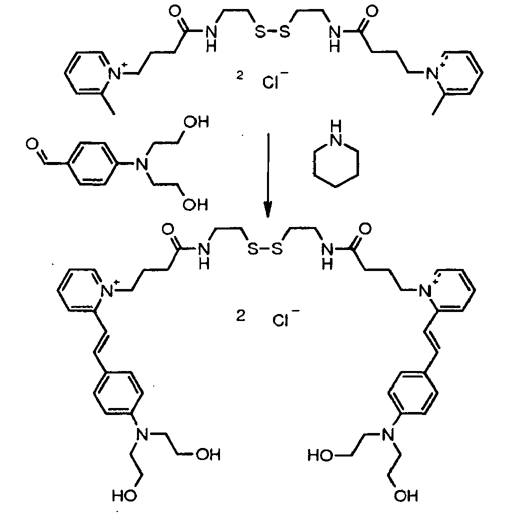

0.37 g de 4-[bis-(2-hydroxy-éthyl)-amino]-benzaldéhyde, 0.47 g de dichlorure de 1,1'-{disulfanediylbis[éthane-2,1-diylimino(4-oxobutane-4,1-diyl)]}bis(4-méthylpyridinium) et 42µL de pipéridine sont ajoutés dans 3 mL méthanol à 70 °C pour 2 h. Le mélange réactionnel est verse dans 25 mL d'acétate d'éthyle et le précipité est filtré puis séché. Une poudre noire est obtenue. Les analyses sont en accord avec la structure du produit attendu. LC/MS gradient ACONH₄ 20mM->CH₃CN 10 min ESI+ m/z=429

### EXEMPLE DE COLORATION

### Exemple 1 : Procédé de coloration - composé [1]

### Préparation d'une composition A

| Composé [1] | 5x10⁻⁴ mol% |
|---|---|
| Alcool Benzylique | 4 g |
| Polyéthylèneglycol 60E | 6 g |
| Hydroxyethylcellulose | 0,7 g |
| Alkylpolyglucoside en solution aqueuse à 65%MA | 4.5 g |
| Eau déminéralisée | qsp 100g |

### Préparation d'une composition B

| | |
|---|---|
| Acide thioglycolique | 1M |
| Hydroxyde de Sodium | qsp pH 8,5 |
| Eau déminéralisée | qsp 100g |

Au moment de l'emploi, on mélange les compositions A (9 mL) et B (1 mL), puis on applique les formules sur mèches de cheveux blancs naturels à 90 % blancs (BN), blancs permanentés (BP) ou cheveux foncés de hauteur de ton 4 (HT4). Le temps de pose est 20 minutes à température ambiante TA.

Après rinçage à l'eau courante on applique un fixateur (Dulcia Vital II®) dilué par 10 avec de l'eau pendant 5 minutes à TA. Après rinçage à l'eau courante et shampooing les mèches sont séchées à l'air on observe un éclaircissement des cheveux foncés ainsi traités : la mèche HT4 est devenue visuellement plus claire que des mèches témoins non traitées. Les mèches de cheveux blancs sont colorées avec des nuances intenses.

### Observations visuelles

Lors du rinçage et des shampooings de l'exemple [1] il n'y a pas de dégorgement visible, la mousse des shampooings et les eaux de rinçage ne sont pratiquement pas colorées.

La couleur observée est conservée sur les BN et BP colorés et l'effet d'éclaircissement reste visible sur les cheveux HT4 shampooinés.

### Test de résistance au shampooings

Les mèches traitées sont lavées par une série de 24 shampooings. On observe que la mousse des shampooings n'est pas ou très peu colorée et que la couleur ne s'affadit pas dans le temps sur les mèches BN et BP. L'effet d'éclaircissement reste également intact.

### Résultats de Réflectance pour évaluer l'éclaircissement optique :

Les performances d'éclaircissement des compositions conformes à l'invention ont été exprimées en fonction de la réflectance des cheveux. Ces réflectances sont comparées à la réflectance d'une mèche de cheveux non traitées de hauteur de ton HT4.

La réflectance est mesurée au moyen d'un appareil spectrophotocolorimètre KONIKA-MINOLTA ®, CM 3600d et après irradiation des cheveux avec une lumière visible dans la gamme de longueurs d'onde allant de 400 à 700 nanomètres.

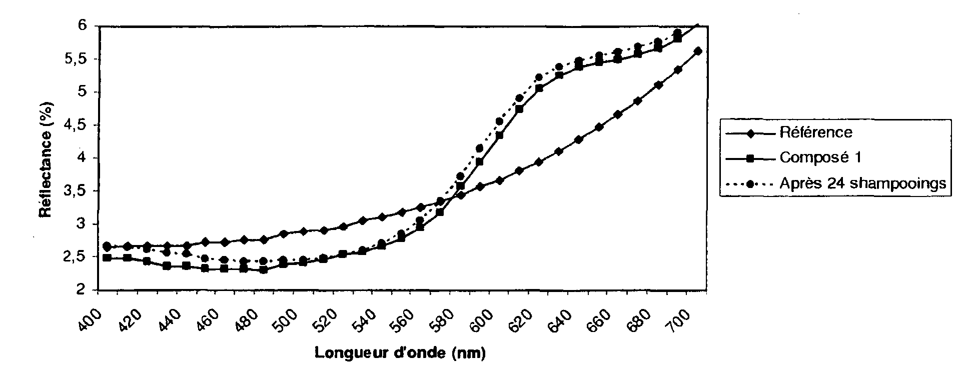

On constate tout d'abord que la réflectance d'une mèche de cheveux traitée avec une composition selon l'invention est supérieure à celle des cheveux non traités. Tout particulièrement la refléctance des mèches traitées avec le colorant [1] est nettement supérieure à celle de la mèche de référence dans la plage de longueur d'ondes supérieure à 580nm. Les mèches traitées par ce composé apparaissent donc plus claires. On constate également que l'effet est parfaitement conservé après 24 shampooings.

### Résultats dans le système L*a*b* pour évaluer la coloration des mèches, BN, BP :

La couleur des mèches été évaluée dans le système L*a*b* au moyen d'un spectrocolorimètre CM 3600D MINOLTA®, (Illuminant D65).

Dans ce système L* a* b*, L* représente la luminosité, a* indique l'axe de couleur vert/rouge et b* l'axe de couleur bleu/jaune. Plus la valeur de L est élevée, plus la couleur est claire ou peu intense. Inversement, plus la valeur de L est faible, plus la couleur est foncée ou très intense. Plus la valeur de a* est élevée plus la nuance est rouge et plus la valeur de b* est élevée plus la nuance est jaune.

La variation de la coloration entre les mèches de cheveux HT4, colorées et lavées est mesurée par (ΔE) selon l'équation suivante : $ΔE = \sqrt{{\left({L}^{*}-{{L}_{o}}^{*}\right)}^{2} + {\left({a}^{*}-{{a}_{o}}^{*}\right)}^{2} + {\left({b}^{*}-{{b}_{o}}^{*}\right)}^{2}}$

Dans cette équation, L*, a* et b* représentent les valeurs mesurées avant coloration et L₀*, a₀* et b₀* représentent les valeurs mesurées avant coloration (ou shampooing).

Plus la valeur de ΔE est importante, plus la différence de couleur entre les mèches HT4 et les mèches colorées est importante.

| | L* | a* | b* | dE* |
|---|---|---|---|---|
| Référence BN | 59,9 | 1,1 | 11,5 | ----- |
| Composé 1 | 36,3 | 38,1 | 23,8 | 45,6 |
| Après 24 shampooings | 39,0 | 37,7 | 21,9 | 43,4 |
| Référence BP | 58,6 | 0,7 | 12,2 | ----- |
| Composé 1 | 32,4 | 40,8 | 26,1 | 49,9 |
| Après 24 shampooings | 31,8 | 40,2 | 23,6 | 49,1 |

Les cheveux blancs naturels et blancs permanentés sont colorées rouge orange vif. Les valeurs évoluent très peu après les shampooings, compte-tenu du nombre de shampoings successifs.

## Revendications

1. Colorant fluorescent de formule (**I**) : 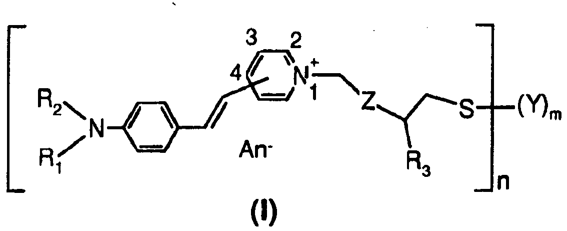 ses sels d'acide organique ou minéral, isomères optiques, isomères géométriques, et les solvates tels que hydrates ;
formule (**I**) dans laquelle :
• **R₁,** représente un groupe alkyle en C₁-C₆ substitué par un ou plusieurs groupes hydroxyle ou -C(O)OR' avec R' représentant un atome d'hydrogène, un groupe alkyle en C₁-C₄ ou alors un groupe -C(O)-O⁻ et dans ce dernier cas un contre ion anionique An⁻ est absent ;
• **R₂,** représente un groupe alkyle en C₁-C₅ éventuellement substitué par un ou plusieurs groupes hydroxyle ;
• ou alors les groupements R₁ et R₂ forment ensemble avec l'atome d'azote qui les portent un radical hétérocyclique saturé, substitué au moins par un groupement hydroxyle, (poly)hydroxy(C₁-C₄)alkyle, et/ou -C(O)OR' avec R' représentant un atome d'hydrogène, un groupe alkyle en C₁-C₄ ou alors un groupe -C(O)-O⁻ et dans ce cas un contre ion anionique An⁻ est absent,
• **R₃** représente un atome d'hydrogène ou un groupement -C(O)OR" avec R" représentant un atome d'hydrogène, un métal alcalin, un groupement alkyle en C₁-C₆ ou alors R₃ représente un groupe -C(O)-O⁻ et dans ce cas un contre-ion anionique An⁻ est absent ;
• **Z** représente un groupe divalent amido -C(O)-N(R)-, -N(R)-C(O)-, ou un groupe divalent alkylène en C₁-C₁₀ interrompu par un groupe amido -C(O)-N(R)-, -N(R)-C(O) avec R représentant un atome d'hydrogène ou un groupe alkyle en C₁-C₆;
• **n** vaut 1 ou 2 ;
• **m** vaut 0 ou 1 ;
• **An** représente un contre ion anionique ;
• **Y** représente : i) un atome d'hydrogène, ii) un métal alcalin, iii) un métal alcalino-terreux, iv) un groupe ammonium : N⁺R^{α}R^{β}R^{γ}R^{δ}, un groupe phosphonium : P⁺A^{α}A^{β}R^{γ}R^{δ} avec R^{α}, R^{β}, R^{γ} et R^{δ}, identiques ou différents, représentant un atome d'hydrogène ou un groupe (C₁-C₄)alkyle, ou v) un groupe protecteur de fonction thiol ;
étant entendu que :
- la liaison entre le cycle pyridinium et la double liaison du groupe styryle est positionnée en position 2 ou 4 du pyridinium ;
- lorsque n=1, m=1 alors Y représente i) à v) tel que défini précédement et lorsque n=2, alors m=0 ;
- lorsque le composé de formule (I) contient d'autres parties cationiques, il se trouve associé à un ou plusieurs contre-ions anioniques permettant d'atteindre l'électroneutralité de la formule (I); et étant entendu que le composé de formule (I) est différents des composés suivants : 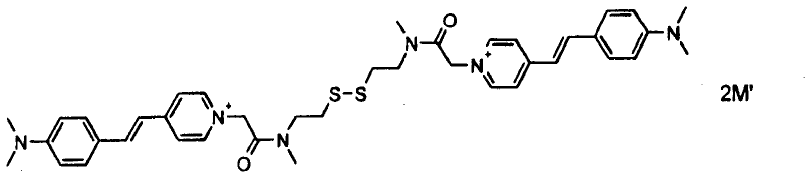 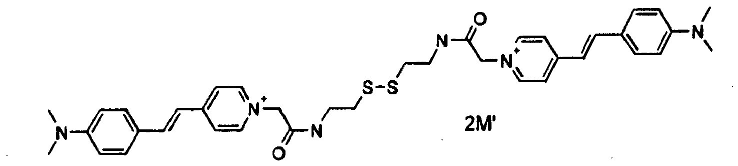 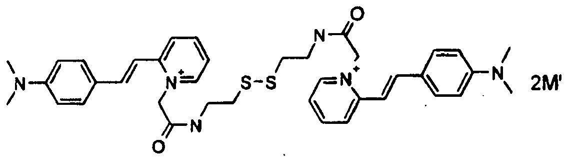 avec M' un contre-ion anionique.

2. Colorant fluorescent thiol de formule **(I)** selon la revendication précédente dans lequel Y représente un atome d'hydrogène, ou un métal alcalin.

3. Colorant fluorescent thiol de formule **(I)** selon la revendication 1 dans lequel Y représente un groupe protecteur.

4. Colorant fluorescent thiol de formule **(I)** selon la revendication précédente dans lequel Y représente un groupe protecteur choisi parmi les radicaux suivants :
▪ (C₁-C₄)alkylcarbonyle ;
▪ (C₁-C₄)alkylthiocarbonyle ;
▪ (C₁-C₄)alcoxycarbonyle ;
▪ (C₁-C₄)alcoxythiocarbonyle ;
▪ (C₁-C₄)alkylthio-thiocarbonyle ;
▪ (di)(C₁-C₄)(alkyl)aminocarbonyle ;
▪ (di)(C₁-C₄)(alkyl)aminothiocarbonyle;
▪ arylcarbonyle ;
▪ aryloxycarbonyle ;
▪ aryl(C₁-C₄)alcoxycarbonyle ;
▪ (di)(C₁-C₄)(alkyl)aminocarbonyle ;
▪ (C₁-C₄)(alkyl)arylaminocarbonyle ;
▪ carboxy ;
▪ SO₃⁻ ; M⁺ avec M⁺ représentant un métal alcalin ou alors An⁻ de la formule (I) et M⁺ sont absents ;
▪ aryle éventuellement substitué ;
▪ hétéroaryle éventuellement substitué ;
▪ hétérocycloalkyle éventuellement substitué, éventuellement cationique,
▪ le groupe suivant : 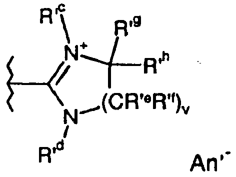 dans lequel A^{'c}, R^{'d}, R^{'e}, R^{'f}, R^{'g} et R^{'h}, identiques ou différents représentent un atome d'hydrogène ou un groupe (C₁-C₄) alkyle, ou alors deux groupe R^{'g} avec R^{'h}, et/ou R^{'e} avec R^{'f} forment un groupe oxo ou thioxo, ou alors R^{'g} avec R^{'e} forment ensemble un cycloalkyle ; et v représente un entier compris inclusivement entre 1 et 3 ; préférentiellement R^{'c} à R^{'h} représentent un atome d'hydrogène ; et An^{'-} représente un contre-ion anionique;
▪ isothiouronium ;
▪ -C(NR^{'e}R^{'c})=N⁺R^{'e}R^{'f}; An^{'-} avec R^{'c}, R^{'d}, R^{'e} et R^{'f}, identiques ou différents représentent un atome d'hydrogène ou un groupe (C₁-C₄)alkyle et An⁻ est tel que défini précédemment ;
▪ isothiourée ;
▪ -C(NR^{,c}R^{'d})=NR^{'e}; An^{'-} avec R^{'c}, R^{'d}, R^{'e} et An⁻ sont tels que définis précédemment ;
▪ (di)aryl(C₁-C₄)alkyle éventuellement substitué ;
▪ (di)hétéroaryl(C₁-C₄)akyle éventuellement substitué;
▪ -CR¹R²R³ avec R¹, R² et R³ identiques ou différents, représentant un atome d'halogène ou un groupe choisi parmi :
i) (C₁-C₄)alkyle ;
ii) (C₁-C₄)alcoxy ;
iii) aryle éventuellement substitué ;
iv) hétéroaryle éventuellement substitué ;
v) P(Z¹)R^{'1}R^{'2}R^{'3} avec R^{'1}, et R^{'2} identiques ou différents représentent un groupe hydroxy, (C₁-C₄)alcoxy ou alkyle, R^{'3} représente un groupe hydroxy ou (C₁-C₄)alcoxy, et Z¹ représente un atome d'oxygène ou de soufre ;
▪ cyclique stériquement encombré ; et
▪ alcoxyalkyle éventuellement substitué.

5. Colorant fluorescent thiol de formule **(I)** selon l'une quelconque des revendications précédentes dans lequel Y représente un métal alcalin ou un groupe protecteur choisi parmi :
➢ (C₁-C₄)alkylcarbonyle ;
➢ arylcarbonyle ;
➢ (C₁-C₄)alkoxycarbonyle ;
➢ aryloxycarbonyle ;
➢ aryl(C₁-C₄)alkoxycarbonyle ;
➢ (di)(C₁-C₄)(alkyl)aminocarbonyle ;
➢ (C₁-C₄)(alkyl)arylaminocarbonyle ;
➢ aryle éventuellement substitué ;
➢ hétéroaryle monocyclique cationique à 5 ou 6 chaînons éventuellement substitué par un ou plusieurs, identiques ou différents, groupes (C₁-C₄)alkyle ;
➢ hétéroaryle bicyclique cationique à 8 à 11 chaînons éventuellement substitué par un ou plusieurs, identiques ou différents, groupes (C₁-C₄)alkyle ;
➢ hétérocycle cationique de formule suivante : 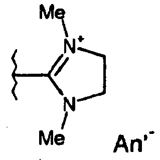
➢ isothiouronium ;
➢ -C(NH₂)=N⁺H₂; An^{'-} ;
➢ isothiourée ;
➢ -C(NH₂)=NH ; et
➢ SO₃⁻ ; M⁺ avec M⁺ représentant un métal alcalin ou alors An⁻ de la formule (I) et M⁺ sont absents.

6. Colorant fluorescent disulfure selon la revendication 1 de formule (**I**) présentant un axe de symétrie C2 entre les deux atomes de soufre du radical central disulfure.

7. Colorant fluorescent selon l'une quelconque des revendications précédentes appartenant une des deux formules (**Ia**) ou (**Ib**) : 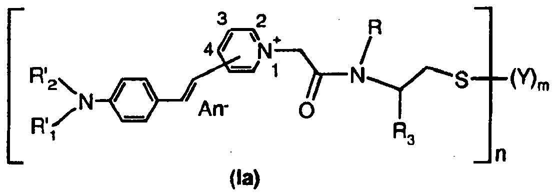 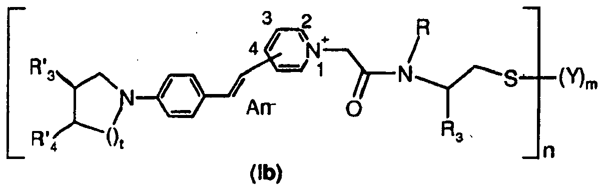 ses sels d'acide organique ou minéral, isomères optiques, isomères géométriques, et les solvates tels que hydrates ;
formules (**Ia**) et (**Ib**) avec :
• **R'**₁, représente un groupe alkyle en C₁-C₄ substitué par un ou plusieurs groupes hydroxyle ou un groupe -C(O)-O⁻;
• **R'**₂, représente un groupe alkyle en C₁-C₄ éventuellement substitué par un ou plusieurs groupes hydroxyle ;
• **R'**₃, et **R'**₄, identiques ou différents, représentent un atome d'hydrogène, un groupe hydroxyle, un groupe hydroxy(C₁-C₄)alkyle ou -C(O)OR' avec R' représentant un atome d'hydrogène, un groupe alkyle en C₁-C₄ ou alors un groupe -C(O)-O⁻ et dans ce cas un contre ion anionique An⁻ est absent, étant entendu qu'un seul de ces deux radicaux **R'**₃ ou **R'**₄ peut représenter un atome d'hydrogène ;
• **R₃** représente un atome d'azote ou un groupement -C(O)OR" avec R" représentant un atome d'hydrogène, un groupe alkyle en C₁-C₄ ou alors R₃ représente un groupe -C(O)-O⁻ et dans ce cas un contre ion anionique An⁻ est absent ;
• **n** vaut 1 ou 2 ;
• **m** vaut 0 ou 1 ;
• **t** vaut 1 ou 2 ;
• **An⁻** représente un contre ion anionique ;
• **Y** représente : i) un atome d'hydrogène ; ii) un métal alcalin ; iii) un métal alcalino-terreux ; iv) un groupe ammonium : N⁺R^{α}R^{β}R^{γ}R^{δ}, An⁻ ou un groupe phosphonium : P⁺R^{α}R^{β}R^{γ}R^{δ}, An⁻ avec R^{α}, R^{β}, R^{γ} et R^{δ}, identiques ou différents, représentant un atome d'hydrogène ou un groupe (C₁-C₄)alkyle et An⁻ est tels que défini précédemment ; ou v) un groupe protecteur de fonction thiol ;
étant entendu que :
- la liaison entre le cycle pyridinium et la double liaison du groupe styryle est positionnée en position 2 ou 4 du pyridinium ;
- lorsque n=1, m=1 alors Y = H ou groupe protecteur de la fonction thiol et lorsque n=2, alors m=0.

8. Colorant fluorescent selon une quelconque des revendications 1, 6 et 7 de formule suivante :
| | |
|---|---|
| 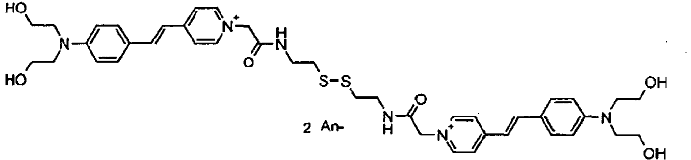 | **1** |
| 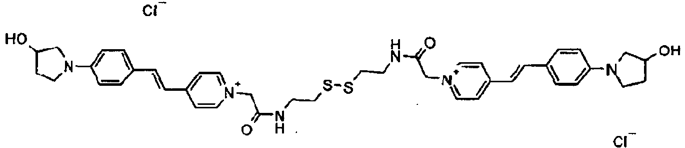 | **2** |
| 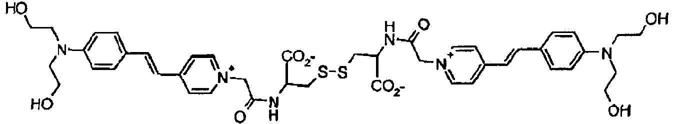 | **3** |
| 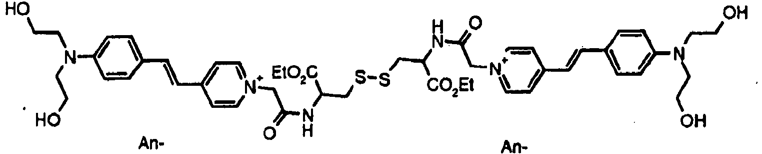 | **4** |
| 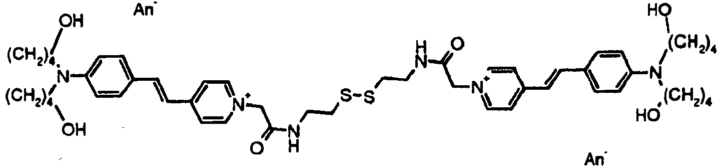 | **5** |
| 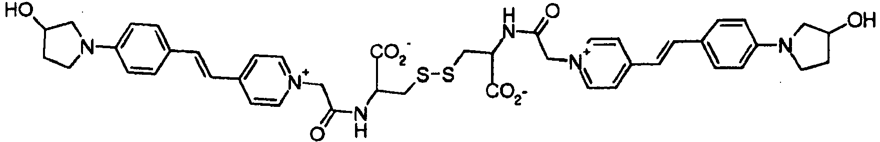 | **6** |
| 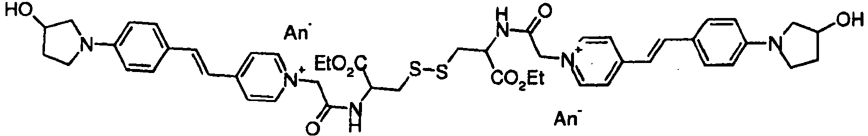 | **7** |
| 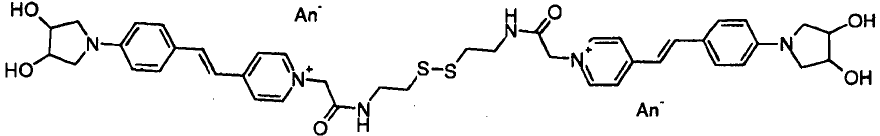 | **8** |
| 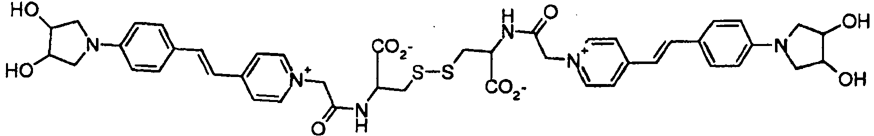 | **9** |
| 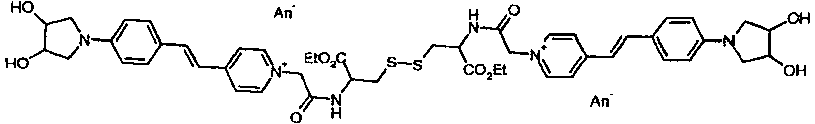 | **10** |
| 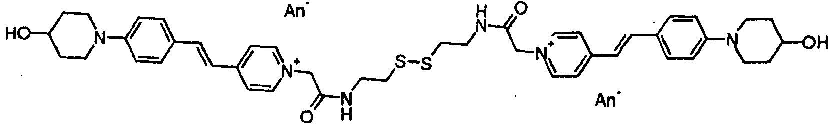 | **11** |
| 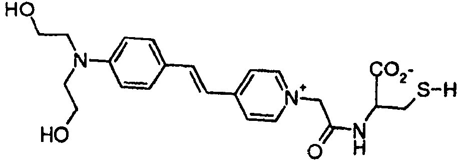 | **12** |
| 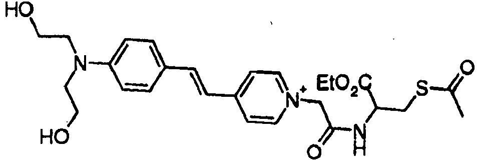 | **13** |
| 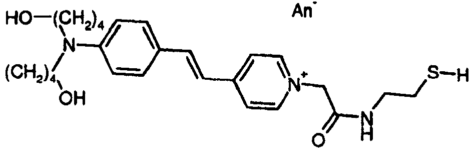 | **14** |
| 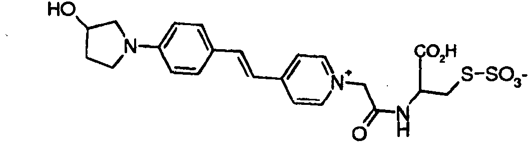 | **15** |
| 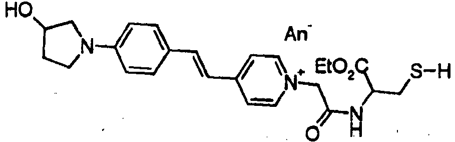 | **16** |
| 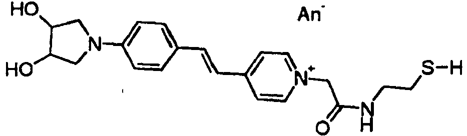 | **17** |
| 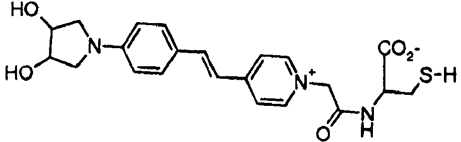 | **18** |
| 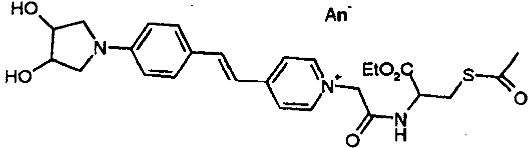 | **19** |
| 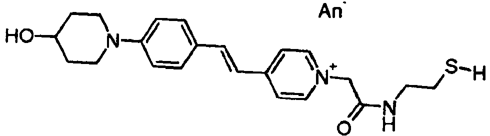 | **20** |
| 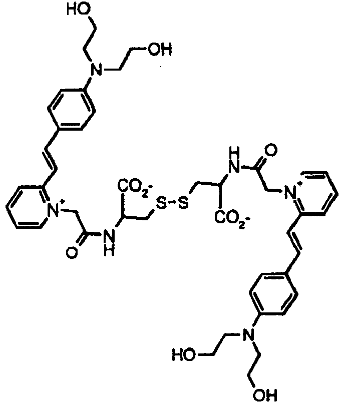 | **21** |
| 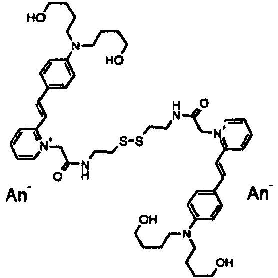 | **22** |
| 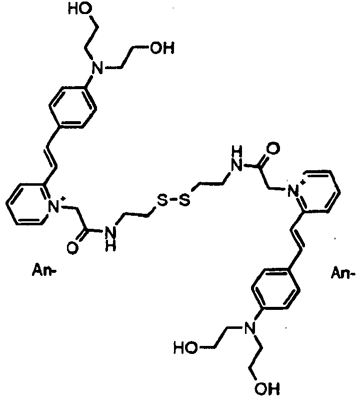 | **23** |
| 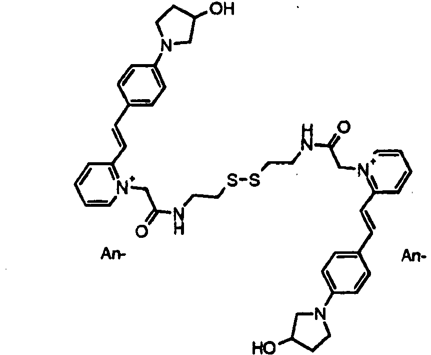 | **24** |
| 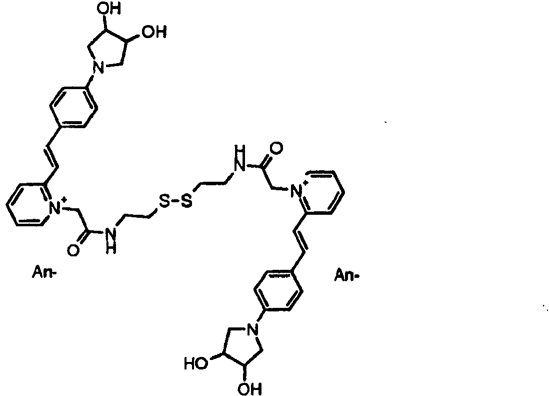 | **25** |
| 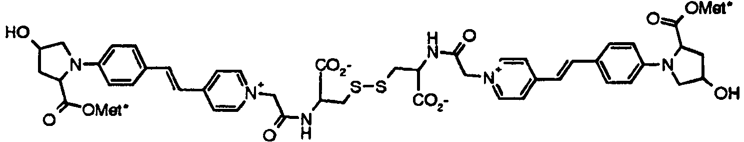 | **26** |
| 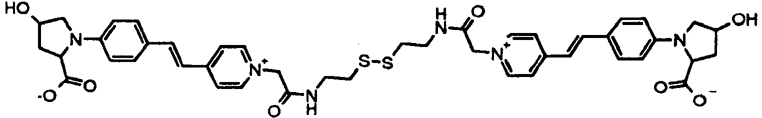 | **27** |
| 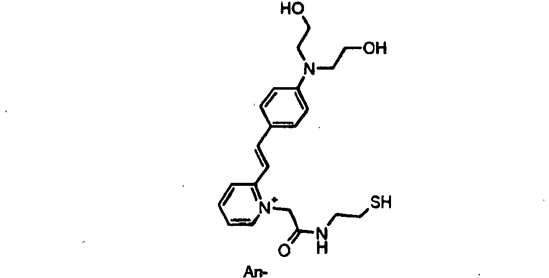 | **28** |
| 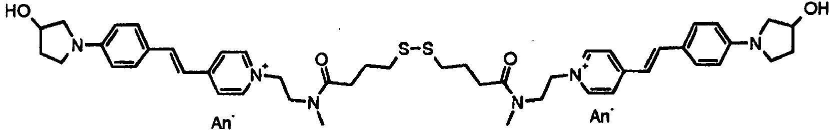 | **29** |
| 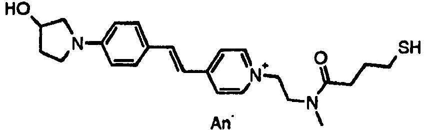 | **30** |
| 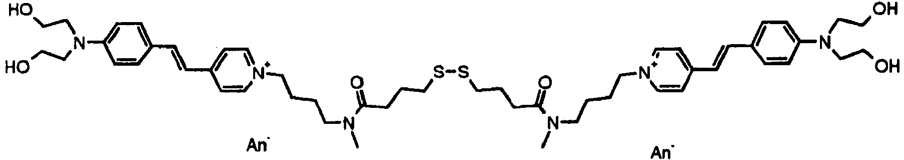 | **31** |
| 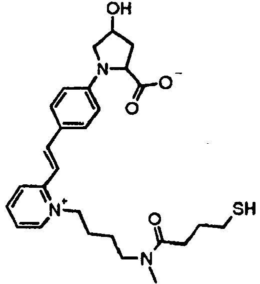 | **32** |
| 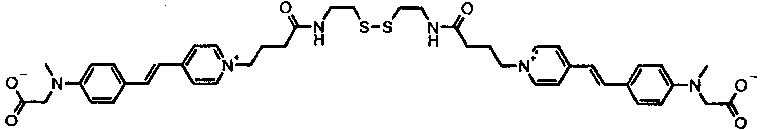 | **33** |
| 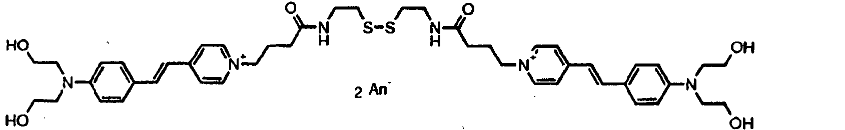 | **34** |
| 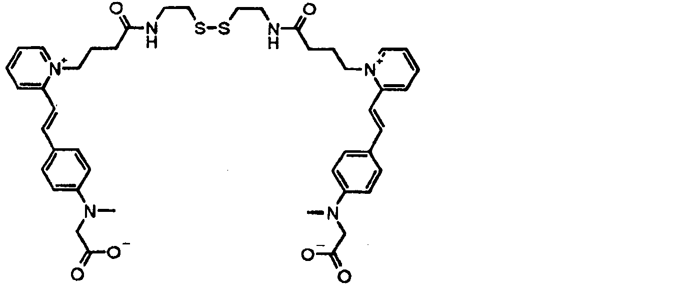 | **35** |
| 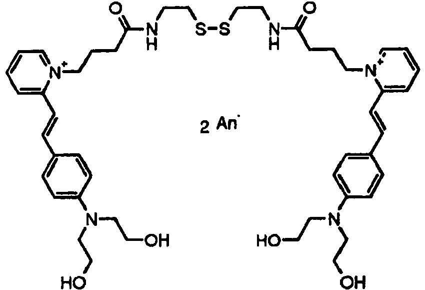 | **36** |
avec An⁻ représentant un contre-ion anionique.

9. Composition tinctoriale comprenant dans un milieu cosmétique approprié, un colorant fluorescent de formule (**I**) tel que défini dans une quelconque des revendications 1 à 8.

10. Composition tinctoriale selon la revendication précédente contenant en outre au moins un agent réducteur.

11. Procédé de coloration de matières kératiniques, dans lequel on applique sur les matières, une composition tinctoriale approprié comprenant au moins un colorant fluorescent de formule (I) tel que défini dans une quelconque des revendications 1 à 8, éventuellement en présence d'un agent réducteur.

12. Procédé de coloration de matières kératiniques selon la revendication précédente **caractérisé en ce que** les matières kératiniques sont des fibres kératiniques foncées possédant une hauteur de ton inférieure ou égale à 6.

13. Dispositif à plusieurs compartiments dans lequel un premier compartiment contient une composition tinctoriale comprenant un colorant fluorescent de formule (**I**) tel que défini aux revendications 1 à 8 et un deuxième compartiment contient un agent réducteur.

14. Utilisation des colorants fluorescents de formule (**I**) tels que définis aux revendications 1 à 8 pour la teinture de fibres kératiniques humaines notamment foncées particulièrement possédant une hauteur de ton inférieure à 6.

15. Utilisation selon la revendication précédente pour l'éclaircissement des fibres kératiniques foncées particulièrement possédant une hauteur de ton inférieure à 6.

## Patentansprüche

1. Fluoreszenzfarbstoff der Formel (I): 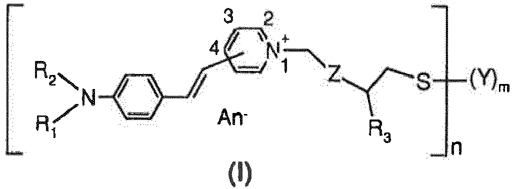 Salze davon mit einer organischen oder anorganischen Säure, optische Isomere davon, geometrische Isomere davon und Solvate wie Hydrate;
wobei in der Formel (I):
• R₁ für eine C₁-C₆-Alkylgruppe, die durch eine oder mehrere Hydroxyl- oder -C(O)OR'-Gruppen substituiert ist, wobei R' für ein Wasserstoffatom oder eine C₁-C₄-Alkylgruppe steht, oder auch eine -C(O)-O⁻-Gruppe steht und in letzterem Fall ein anionisches Gegenion An⁻fehlt;
• R₂ für eine C₁-C₆-Alkylgruppe, die gegebenenfalls durch eine oder mehrere Hydroxylgruppen substituiert ist, steht;
• oder auch die Gruppen R₁ und R₂ zusammen mit dem Stickstoffatom, das sie trägt, einen gesättigten heterocyclischen Rest bilden, der durch mindestens eine Hydroxyl-, (Poly)hydroxy(C₁-C₄)alkyl- und/oder -C(O)-OR'-Gruppe substituiert ist, wobei R' für ein Wasserstoffatom, eine C₁-C₄-Alkylgruppe oder auch eine -C(O)-O⁻-Gruppe steht und in diesem Fall ein anionisches Gegenion An⁻ fehlt;
• R₃ für ein Wasserstoffatom oder eine -C(O)OR"-Gruppe steht, wobei R" für ein Wasserstoffatom, ein Alkalimetall oder eine C₁-C₆-Alkylgruppe steht, oder auch R₃ für eine -C(O)-O⁻-Gruppe steht und in diesem Fall ein anionisches Gegenion An⁻ fehlt;
• Z für eine zweiwertige -C(O)-N(R)- oder -N(R)-C(O)-Amidogruppe oder eine zweiwertige C₁-C₁₀-Alkylengruppe, die durch eine -C(O)-N(R)- oder -N(R)-C(O)-Amidogruppe unterbrochen ist, steht, wobei R für ein Wasserstoffatom oder eine C₁-C₆-Alkylgruppe steht;
• n für 1 oder 2 steht;
• m für 0 oder 1 steht;
• An⁻ für ein anionisches Gegenion steht;
• Y für Folgendes steht: i) ein Wasserstoffatom, ii) ein Alkalimetall, iii) ein Erdalkalimetall, iv) eine Ammoniumgruppe: N⁺R^{α}R^{β}R^{γ}R^{δ} oder eine Phosphoniumgruppe: P⁺R^{α}R^{β}R^{γ}R^{δ}, wobei R^{α}, R^{β}, R^{γ} und R^{δ} gleich oder verschieden sind und für ein Wasserstoffatom oder eine (C₁-C₄)Alkylgruppe stehen; oder v) eine Schutzgruppe für die Thiolfunktion;
mit den Maßgaben, dass:
- die Bindung zwischen dem Pyridiniumring und der Doppelbindung der Styrylgruppe in Position 2 oder 4 des Pyridiniums steht;
- wenn n=1 und m=1, dann Y für i) bis v) gemäß obiger Definition steht und wenn n=2, dann m=0;
- die Verbindung der Formel (I) dann, wenn sie andere kationische Teile enthält, mit einem oder mehreren anionischen Gegenionen assoziiert ist, so dass die Elektroneutralität der Formel (I) gewährleistet ist; und
mit der Maßgabe, dass die Verbindung der Formel (I) von den folgenden Verbindungen verschieden ist: 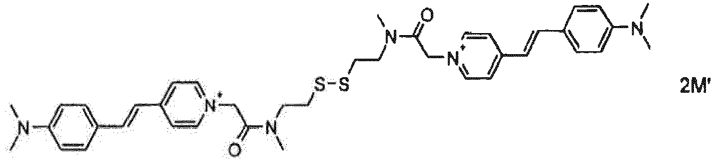 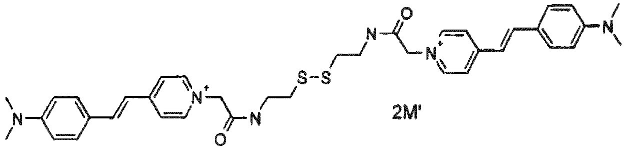 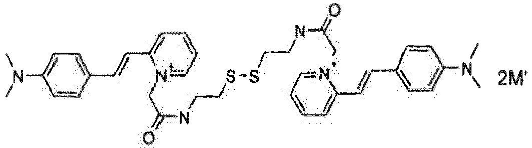 wobei M' für ein anionisches Gegenion steht.

2. Thiol-Fluoreszenzfarbstoff der Formel (I) nach dem vorhergehenden Anspruch, worin Y für ein Wasserstoffatom oder ein Alkalimetall steht.

3. Thiol-Fluoreszenzfarbstoff der Formel (I) nach Anspruch 1, worin Y für eine Schutzgruppe steht.

4. Thiol-Fluoreszenzfarbstoff der Formel (I) nach dem vorhergehenden Anspruch, worin Y für eine Schutzgruppe steht, die unter den folgenden Resten ausgewählt ist:
▪ (C₁-C₄) Alkylcarbonyl;
▪ (C₁-C₄)Alkylthiocarbonyl;
▪ (C₁-C₄)Alkoxycarbonyl;
▪ (C₁-C₄)Alkoxythiocarbonyl;
▪ (C₁-C₄)Alkylthiothiocarbonyl;
▪ (Di) (C₁-C₄) (alkyl)aminocarbonyl;
▪ (Di)(C₁-C₄)(alkyl)aminothiocarbonyl;
▪ Arylcarbonyl;
▪ Aryloxycarbonyl;
▪ Aryl(C1-C4)alkoxycarbonyl;
▪ (Di) (C₁-C₄) (alkyl)aminocarbonyl;
▪ (C₁-C₄) (Alkyl) arylaminocarbonyl;
▪ Carboxyl;
▪ SO₃⁻; M⁺, wobei M⁺ für ein Alkalimetall steht oder auch An⁻ der Formel (I) und M⁺ fehlen;
▪ gegebenenfalls substituiertem Aryl;
▪ gegebenenfalls substituiertem Heteroaryl;
▪ gegebenenfalls kationischem, gegebenenfalls substituiertem Heterocycloalkyl;
▪ der folgenden Gruppe: 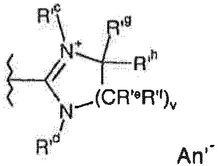 worin R^{'c}, R^{'d}, R^{'e}, R^{'f}, R^{'g} und R^{'h} gleich oder verschieden sind und für ein Wasserstoffatom oder eine (C₁-C₄)Alkylgruppe stehen oder auch zwei Gruppen R^{'g} mit R^{'h} und/oder R^{'e} mit R^{'f} eine Oxo- oder Thioxogruppe bilden oder auch R^{'g} mit R^{'e} zusammen ein Cycloalkyl bilden und v für eine ganze Zahl zwischen 1 und 3 einschließlich steht; R^{'c} bis R^{'h} vorzugsweise für ein Wasserstoffatom stehen; und An^{'-} für ein anionisches Gegenion steht;
▪ Isothiouronium;
▪ -C(NR^{'c}R^{'d})=N⁺R^{,e}R^{,f}; An^{'-}, wobei R^{'c}, R^{'d}, R^{'e}, und R^{'f} gleich oder verschieden sind und für ein Wasserstoffatom oder eine (C₁-C₄)Alkylgruppe stehen und An⁻ die oben angegebene Bedeutung besitzt;
▪ Isothioharnstoff;
▪ -C(NR^{'c}R^{'d})=NR^{'e}; An^{'-} wobei R^{'c}, R^{'d} und R^{'e} und An⁻ die oben angegebene Bedeutung besitzen;
▪ gegebenenfalls substituiertem (Di)aryl(C₁-C₄)-alkyl;
▪ gegebenenfalls substituiertem (Di)heteroaryl(C₁-C₄)alkyl;
▪ -CR¹R²R³, wobei R¹, R² und R³ gleich oder verschieden sind und für ein Halogenatom oder eine unter:
i) (C₁-C₄)Alkyl;
ii) (C₁-C₄)Alkoxy;
iii) gegebenenfalls substituiertem Aryl;
iv) gegebenenfalls substituiertem Heteroaryl;
v) P(Z¹)R^{'1}R^{'2}R^{'3}, wobei R^{'1} und R^{'2} gleich oder verschieden sind und für eine Hydroxyl-, (C₁-C₄)Alkoxy- oder Alkylgruppe stehen, R^{'3} für eine Hydroxyl- oder (C₁-C₄)Alkoxygruppe steht und Z¹ für ein Sauerstoff- oder Schwefelatom steht;
ausgewählte Gruppe stehen;
▪ einer sterisch gehinderten cyclischen Gruppe; und
▪ gegebenenfalls substituiertem Alkoxyalkyl.

5. Thiol-Fluoreszenzfarbstoff der Formel (I) nach einem der vorhergehenden Ansprüche, worin Y für ein Alkalimetall oder eine unter:
➢ (C₁-C₄)Alkylcarbonyl;
➢ Arylcarbonyl;
➢ (C₁-C₄)Alkoxycarbonyl;
➢ Aryloxycarbonyl;
➢ Aryl (C₁-C₄)alkoxycarbonyl;
➢ (Di) (C₁-C₄) (alkyl)aminocarbonyl;
➢ (C₁-C₄)(Alkyl)arylaminocarbonyl ;
➢ gegebenenfalls substituiertem Aryl;
➢ 5- oder 6-gliedrigem kationischem monocyclischem Heteroaryl, das gegebenenfalls durch eine oder mehrere gleiche oder verschiedene (C₁-C₄)-Alkylgruppen substituiert ist;
➢ 8- bis 11-gliedrigem kationischem bicyclischem Heteroaryl, das gegebenenfalls durch eine oder mehrere gleiche oder verschiedene (C₁-C₄)-Alkylgruppen substituiert ist;
➢ dem kationischen Heterocyclus der nachstehenden Formel: 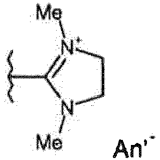
➢ Isothiouronium;
➢ -C (NH₂)=N⁺H₂; An^{'-};
➢ Isothioharnstoff;
➢ -C(NH₂)=NH; und
➢ SO₃⁻; M⁺, wobei M⁺ für ein Alkalimetall steht oder auch An⁻ der Formel (I) und M⁺ fehlen;
ausgewählte Schutzgruppe steht.

6. Disulfid-Fluoreszenzfarbstoff nach Anspruch 1 der Formel (I) mit einer C2-Symmetrieachse zwischen den beiden Schwefelatomen des zentralen Disulfidrests.

7. Fluoreszenzfarbstoff nach einem der vorhergehenden Ansprüche, der zu einer der Formeln (Ia) oder (Ib) gehört: 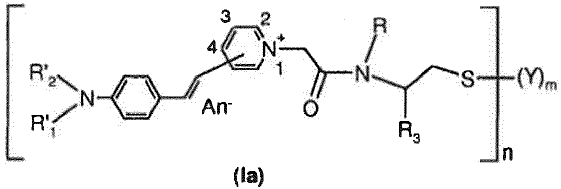 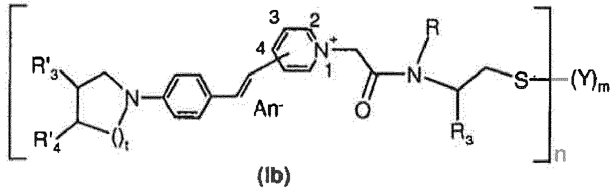 Salze davon mit einer organischen oder anorganischen Säure, optische Isomere davon, geometrische Isomere davon und Solvate wie Hydrate;
wobei in den Formeln (Ia) und (Ib):
• R'₁ für eine C₁-C₄-Alkylgruppe, die durch eine oder mehrere Hydroxylgruppen oder eine -C(O)O⁻-Gruppe substituiert ist, steht;
• R'₂ für eine C₁-C₄-Alkylgruppe, die gegebenenfalls durch eine oder mehrere Hydroxylgruppen substituiert ist, steht;
• R'₃ und R'₄ gleich oder verschieden sind und für ein Wasserstoffatom, eine Hydroxylgruppe, eine Hydroxy(C₁-C₄)alkylgruppe oder eine -C(O)OR'-Gruppe stehen, wobei R' für ein Wasserstoffatom, eine C₁-C₄-Alkylgruppe oder auch eine -C(O)-O-⁻Gruppe steht und in diesem Fall ein anionisches Gegenion An⁻ fehlt, mit der Maßgabe, dass nur einer dieser beiden Reste R'₃ oder R'₄ für ein Wasserstoffatom stehen kann;
• R₃ für ein Stickstoffatom oder eine -C(O)OR"-Gruppe steht, wobei R" für ein Wasserstoffatom oder eine C₁-C₄-Alkylgruppe steht, oder auch R₃ für eine -C(O)-O⁻-Gruppe steht und in diesem Fall ein anionisches Gegenion An⁻ fehlt;
• n für 1 oder 2 steht;
• m für 0 oder 1 steht;
• t für 1 oder 2 steht;
• An⁻ für ein anionisches Gegenion steht;
• Y für Folgendes steht: i) ein Wasserstoffatom, ii) ein Alkalimetall, iii) ein Erdalkalimetall, iv) eine Ammoniumgruppe: N⁺R^{α}R^{β}R^{γ}R^{δ}, An oder eine Phosphoniumgruppe: P⁺R^{α}R^{β}R^{γ}R^{δ}, An⁻, wobei R^{α}, R^{β}, R^{γ} und R^{δ} gleich oder verschieden sind und für ein Wasserstoffatom oder eine (C₁-C₄)Alkyl-gruppe stehen und An⁻ die oben angegebene Bedeutung besitzt; oder v) eine Schutzgruppe für die Thiolfunktion;
mit den Maßgaben, dass:
- die Bindung zwischen dem Pyridiniumring und der Doppelbindung der Styrylgruppe in Position 2 oder 4 des Pyridiniums steht;
- wenn n=1 und m=1, dann Y =H oder eine Schutzgruppe der Thiolfunktion und wenn n=2, dann m=0.

8. Fluoreszenzfarbstoff nach einem der Ansprüche 1, 6 und 7 der folgenden Formel:
| | |
|---|---|
| 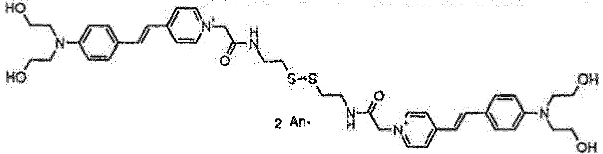 | **1** |
| 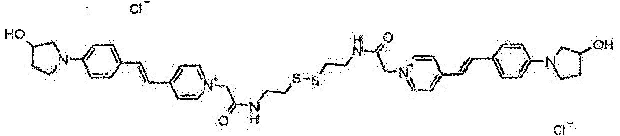 | **2** |
| 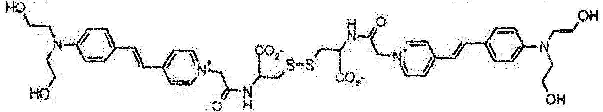 | **3** |
| 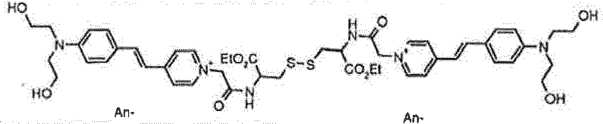 | **4** |
| 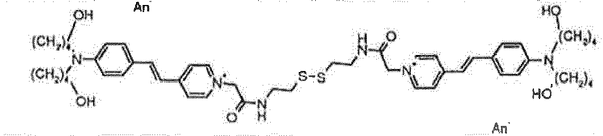 | **5** |
| 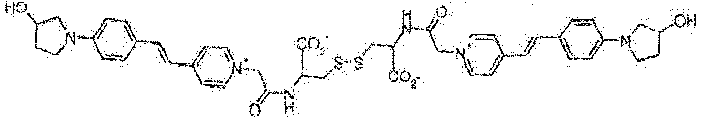 | **6** |
| 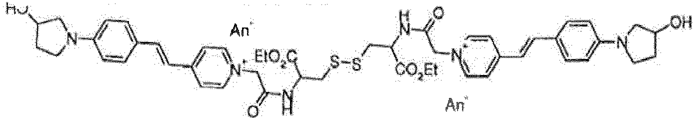 | **7** |
| 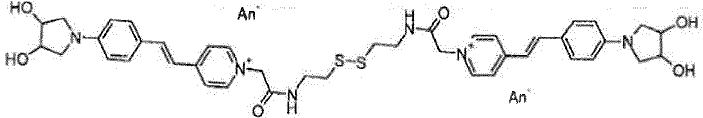 | **8** |
| 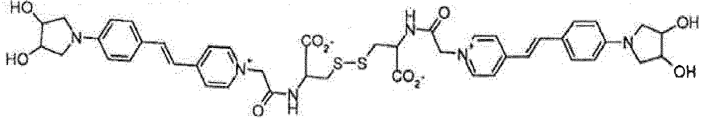 | **9** |
| 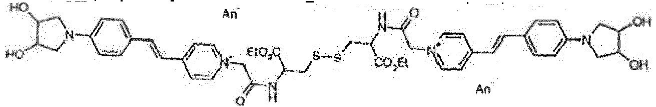 | **10** |
| 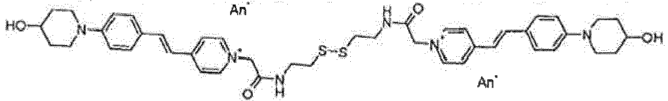 | **11** |
| 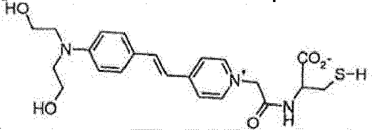 | **12** |
| 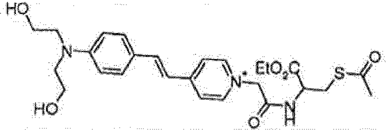 | **13** |
| 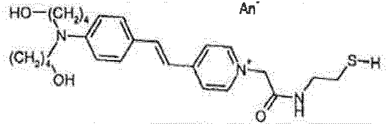 | **14** |
| 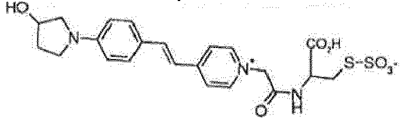 | **15** |
| 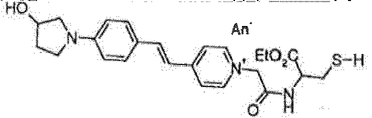 | **16** |
| 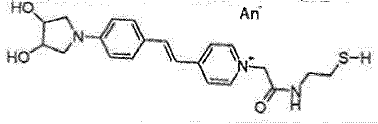 | **17** |
| 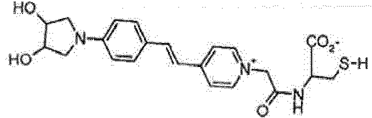 | **18** |
| 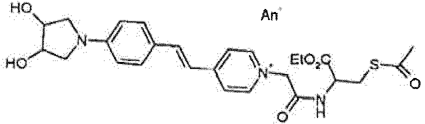 | **19** |
| 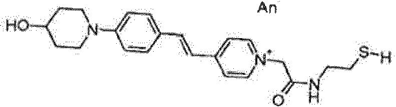 | **20** |
| 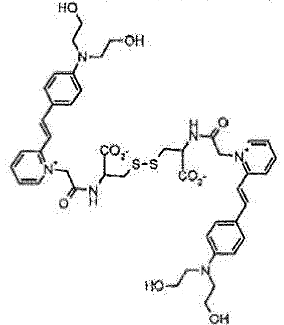 | **21** |
| 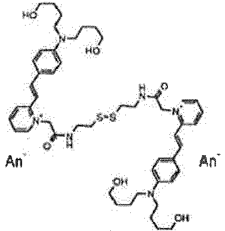 | **22** |
| 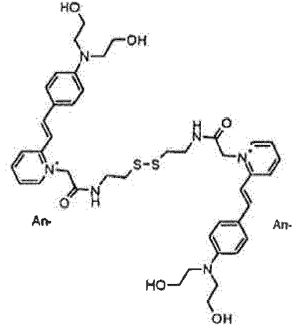 | **23** |
| 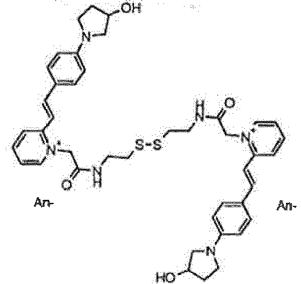 | **24** |
| 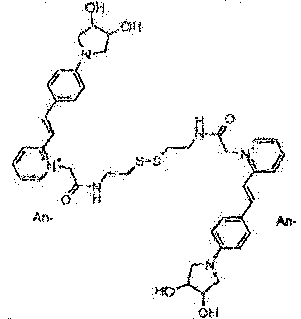 | **25** |
| 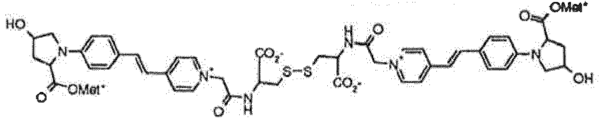 | **26** |
| 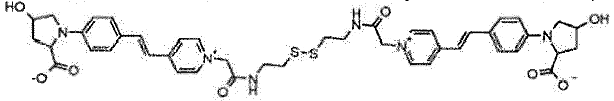 | **27** |
| 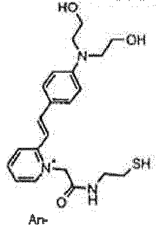 | **28** |
| 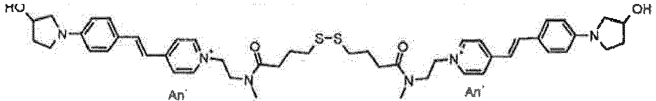 | **29** |
| 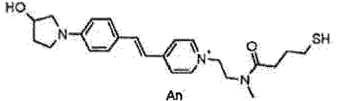 | **30** |
| 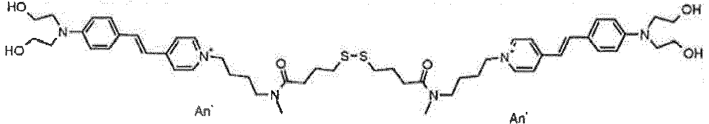 | **31** |
| 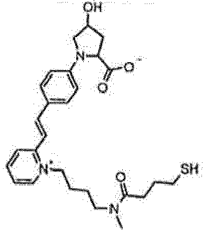 | **32** |
| 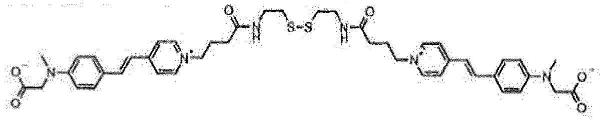 | **33** |
| 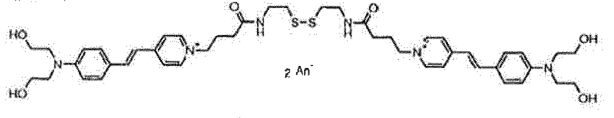 | **34** |
| 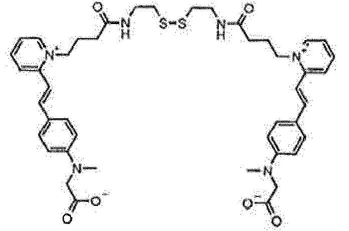 | **35** |
| 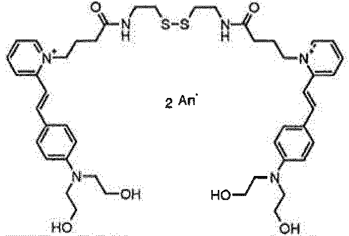 | **36** |
wobei An⁻ für ein anionisches Gegenion steht.

9. Färbezusammensetzung, die in einem geeigneten kosmetischen Medium einen Fluoreszenzfarbstoff der Formel (I) gemäß einem der Ansprüche 1 bis 8 umfasst.

10. Färbezusammensetzung nach dem vorhergehenden Anspruch, die außerdem mindestens ein Reduktionsmittel umfasst.

11. Verfahren zum Färben von Keratinmaterialien, bei dem man auf die Materialien eine geeignete Färbezusammensetzung, die mindestens einen Fluoreszenzfarbstoff der Formel (I) gemäß einem der Ansprüche 1 bis 8 umfasst, aufbringt, gegebenenfalls in Gegenwart eines Reduktionsmittels.

12. Verfahren zum Färben von Keratinmaterialien nach dem vorhergehenden Anspruch, **dadurch gekennzeichnet, dass** es sich bei den Keratinmaterialien um dunkle Keratinfasern mit einer Tonhöhe kleiner gleich 6 handelt.

13. Vorrichtung mit mehreren Kompartimenten, in der ein erstes Kompartiment eine Färbezusammensetzung, die einen Fluoreszenzfarbstoff der Formel (I) gemäß einem der Ansprüche 1 bis 8 umfasst, enthält und ein zweites Kompartiment ein Reduktionsmittel enthält.

14. Verwendung der Fluoreszenzfarbstoffe der Formel (I) gemäß einem der Ansprüche 1 bis 8 zum Färben von menschlichen Keratinfasern, insbesondere dunklen Keratinfasern, speziell solchen mit einer Tonhöhe kleiner 6.

15. Verwendung nach dem vorhergehenden Anspruch zur Aufhellung von dunklen Keratinfasern, insbesondere solchen mit einer Tonhöhe kleiner 6.

## Claims

1. Fluorescent dye of formula (**I**): 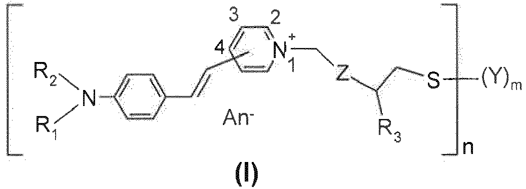 the organic or mineral acid salts, optical isomers and geometric isomers thereof, and the solvates such as hydrates;
in which formula (**I**):
• **R₁** represents a C₁-C₆ alkyl group substituted with one or more hydroxyl groups or -C(O)OR' groups, with R' representing a hydrogen atom, a C₁-C₄ alkyl group or else a -C(O)-O⁻ group and, in the latter case, an anionic counterion An⁻ is absent;
• **R₂** represents a C₁-C₆ alkyl group optionally substituted with one or more hydroxyl groups;
• or else the groups R₁ and R₂ form, together with the nitrogen atom which bears them, a saturated heterocyclic radical substituted at least with a hydroxyl, (poly) hydroxy (C₁-C₄) alkyl, and/or -C(O)OR' group, with R' representing a hydrogen atom, a C₁-C₄ alkyl group or else a -C(O)-O⁻ group and, in this case, an anionic counterion An⁻ is absent;
• **R₃** represents a hydrogen atom or a -C(O)OR" group, with R" representing a hydrogen atom, an alkali metal or a C₁-C₆ alkyl group, or else R₃ represents a -C(O)-O⁻ group and, in this case, an anionic counterion An⁻ is absent;
• **Z** represents a divalent amido -C(O)-N(R)- or -N(R)-C(O)- group, or a divalent C₁-C₁₀ alkylene group interrupted with an amido -C(O)-N(R)- or -N(R)-C(O)- group, with R representing a hydrogen atom or a C₁-C₆ alkyl group;
• **n** is 1 or 2;
• **m** is 0 or 1;
• **An⁻** represents an anionic counterion;
• **Y** represents: i) a hydrogen atom, ii) an alkali metal, iii) an alkaline earth metal, iv) an ammonium group: N⁺R^{α}R^{β}R^{γ}R^{δ}, a phosphonium group: P⁺R^{α}R^{β}R^{γ}R^{δ} with R^{α}, R^{β}, R^{γ} and R^{δ}, which may be identical or different, representing a hydrogen atom or a (C₁-C₄)alkyl group, or v) a thiol-function-protecting group;
it being understood that:
- the bond between the pyridinium ring and the double bond of the styryl group is positioned in the 2- or 4-position with respect to the pyridinium;
- when n=1, m=1, then Y represents i) to v) as defined above and when n=2, then m=0;
- when the compound of formula **(I)** contains other cationic parts, it is associated with one or more anionic counterions which allow formula **(I)** to achieve electroneutrality, and
it being understood that the compound of formula (I) is different from the following compounds: 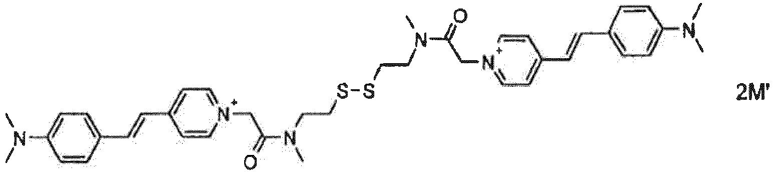 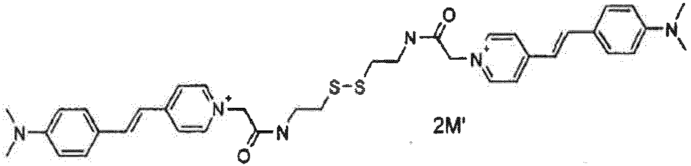 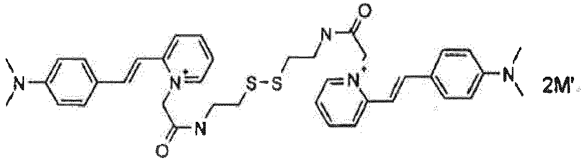 with M' being an anionic counterion.

2. Fluorescent thiol dye of formula **(I)** according to the preceding claim, in which Y represents a hydrogen atom or an alkali metal.

3. Fluorescent thiol dye of formula **(I)** according to Claim 1, in which Y represents a protecting group.

4. Fluorescent thiol dye of formula **(I)** according to the preceding claim, in which Y represents a protecting group chosen from the following radicals:
• (C₁-C₄)alkylcarbonyl;
• (C₁-C₄)alkylthiocarbonyl;
• (C₁-C₄)alkoxycarbonyl;
• (C₁-C₄)alkoxythiocarbonyl;
• (C₁-C₄)alkylthiothiocarbonyl;
• (di) (C₁-C₄) (alkyl)aminocarbonyl;
• (di) (C₁-C₄) (alkyl)aminothiocarbonyl;
• arylcarbonyl;
• aryloxycarbonyl;
• aryl(C₁-C₄)alkoxycarbonyl;
• (di) (C₁-C₄) (alkyl)aminocarbonyl;
• (C₁-C₄) (alkyl)arylaminocarbonyl;
• carboxyl;
• SO₃⁻; M⁺ with M⁺ representing an alkali metal or else An⁻ of the formula (I) and M⁺ are absent;
• optionally substituted aryl;
• optionally substituted heteroaryl;
• optionally cationic, optionally substituted heterocycloalkyl,
• the following group: 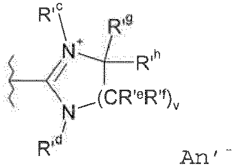 in which R^{'c}, R^{'d}, R^{'e}, R^{'f}, R^{'g} and R^{'h}, which may be identical or different, represent a hydrogen atom or a (C₁-C₄) alkyl group, or else two groups R^{'g} with R^{'h}, and/or R^{'e} with R^{'f} form an oxo or thioxo group, or else R^{'g} with R^{'e} together form a cycloalkyl; and v represents an integer between 1 and 3 inclusive; preferably R^{'c} to R^{'h} represent a hydrogen atom; and An^{'-} represents an anionic counterion;
▪ isothiouronium;
▪ -C(NR^{'c}R^{'d})=N⁺R^{'e}R^{'f}; An^{,-} with R^{'c}, R^{'d}, R^{'e} and R^{'f}, which may be identical or different, representing a hydrogen atom or a (C₁-C₄)alkyl group and An⁻ being as defined above;
▪ isothiourea;
▪ -C(NR^{'c}R^{'d})=NR^{'e}; An' with R^{'c}, R^{'d}, R^{'e} and An⁻being as defined above;
▪ optionally substituted (di)aryl(C₁-C₄)alkyl;
▪ optionally substituted (di)heteroaryl(C₁-C₄)alkyl;
▪ -CR¹R²R³ with R¹, R² and R³, which may be identical or different, representing a halogen atom or a group chosen from:
i) (C₁-C₄)alkyl;
ii) (C₁-C₄)alkoxy;
iii) optionally substituted aryl;
iv) optionally substituted heteroaryl;
v) P(Z¹)R^{'1}R^{'2}R^{'3} with R^{'1} and R^{'2}, which may be identical or different, representing a hydroxyl, (C₁-C₄)alkoxy or alkyl group, R^{'3} representing a hydroxyl or (C₁-C₄)alkoxy group, and Z¹ representing an oxygen or sulfur atom;
▪ a sterically hindered cyclic group; and
▪ optionally substituted alkoxyalkyl.

5. Fluorescent thiol dye of formula (I) according to any one of the preceding claims, in which Y represents an alkali metal or a protecting group chosen from:
➢ (C₁-C₄)alkylcarbonyl;
➢ arylcarbonyl;
➢ (C₁-C₄)alkoxycarbonyl;
➢ aryloxycarbonyl;
➢ aryl (C₁-C₄)alkoxycarbonyl;
➢ (di)(C₁-C₄)(alkyl)aminocarbonyl;
➢ (C₁-C₄)(alkyl)arylaminocarbonyl;
➢ optionally substituted aryl;
➢ 5- or 6-membered cationic monocyclic heteroaryl optionally substituted with one or more (C₁-C₄)alkyl groups which may be identical or different;
➢ 8- to 11-membered cationic bicyclic heteroaryl optionally substituted with one or more (C₁-C₄)alkyl groups which may be identical or different;
➢ cationic heterocycle of the following formula: 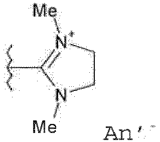
➢ isothiouronium;
➢ -C(NH₂)=N⁺H₂; An^{'-};
➢ isothiourea;
➢ -C(NH₂)=NH; and
➢ SO₃⁻; M⁺ with M⁺ representing an alkali metal or else An⁻ of the formula (I) and M⁺ are absent.

6. Disulfide fluorescent dye according to Claim 1, of formula (**I**), having a C2 axis of symmetry between the two sulfur atoms of the central disulfide radical.

7. Fluorescent dye according to any one of the preceding claims, belonging to one of the two formulae (**Ia**) and (**Ib**): 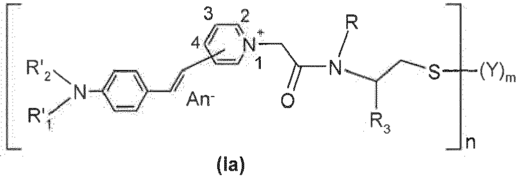 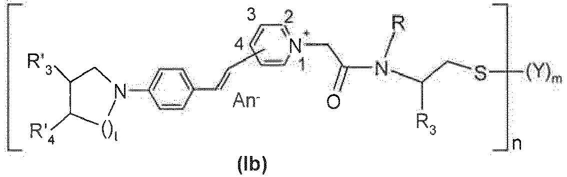 the organic or mineral acid salts, optical isomers and geometric isomers thereof, and the solvates such as hydrates;
in which formulae (**Ia**) and (**Ib**):
• **R'**₁ represents a C₁-C₄ alkyl group substituted with one or more hydroxyl groups or a -C(O)-O⁻ group;
• **R'**₂ represents a C₁-C₄ alkyl group optionally substituted with one or more hydroxyl groups;
• **R'**₃ and **R'**₄, which may be identical or different, represent a hydrogen atom, a hydroxyl group, a hydroxy(C₁-C₄)alkyl group or a -C(O)OR' group, with R' representing a hydrogen atom or a C₁-C₄ alkyl group, or else a -C(O)-O⁻ group and, in this case, an anionic counterion An⁻ is absent, it being understood that just one of these two radicals **R'**₃ or **R'**₄ can represent a hydrogen atom;
• **R₃** represents a nitrogen atom or a -C(O)OR'' group with R" representing a hydrogen atom or a C₁-C₄ alkyl group, or else R₃ represents a -C(O)-O⁻ group and, in this case, an anionic counterion An⁻ is absent;
• **n** is 1 or 2;
• **m** is 0 or 1;
• **t** is 1 or 2;
• **An⁻** represents an anionic counterion;
• **Y** represents: i) a hydrogen atom; ii) an alkali metal; iii) an alkaline earth metal; iv) an ammonium group: N⁺R^{α}R^{β}R^{γ}R^{δ}, An⁻ or a phosphonium group: P⁺R^{α}R^{β}R^{γ}R^{δ}, An⁻ with R^{α}, R^{β}, R^{γ} and R^{δ}, which may be identical or different, representing a hydrogen atom or a (C₁-C₄)alkyl group, and An⁻ as defined above; or v) a thiol-function-protecting group;
it being understood that:
- the bond between the pyridinium ring and the double bond of the styryl group is positioned in the 2- or 4-position with respect to the pyridinium;
- when n=1, m=1, then Y = H or a thiol-function-protecting group and when n=2, then m=0.

8. Fluorescent dye according to any one of Claims 1, 6 and 7, of the following formula:
| | |
|---|---|
| 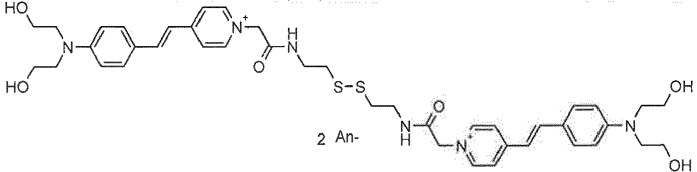 | **1** |
| 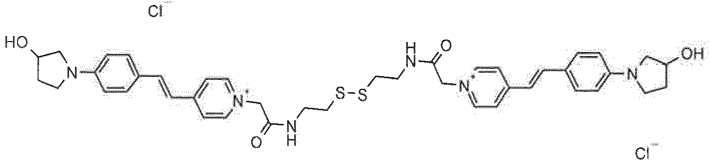 | **2** |
| 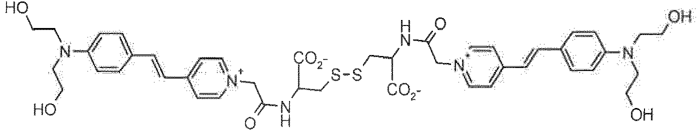 | **3** |
| 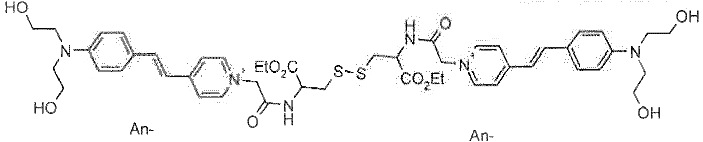 | **4** |
| 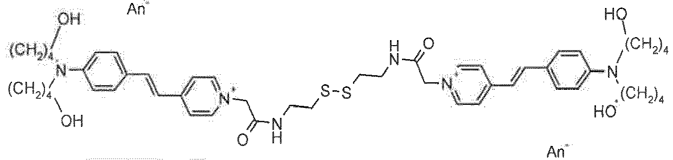 | **5** |
| 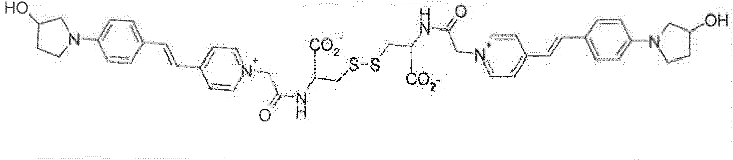 | **6** |
| 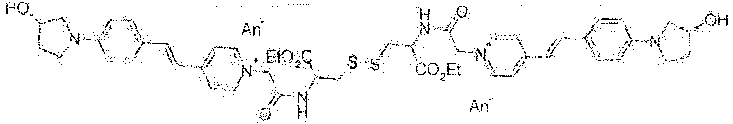 | **7** |
| 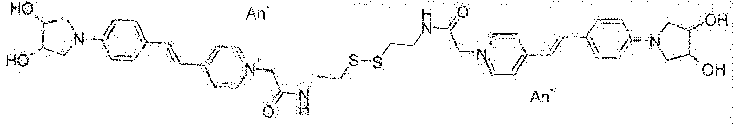 | **8** |
| 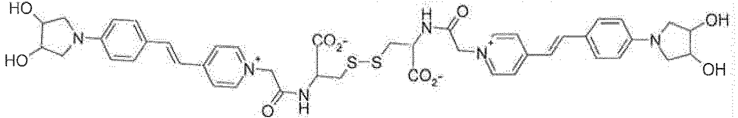 | **9** |
| 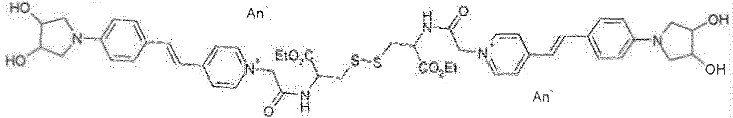 | **10** |
| 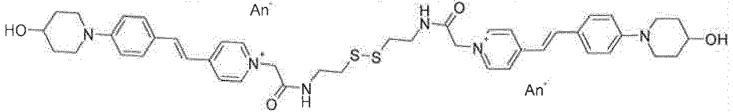 | **11** |
| 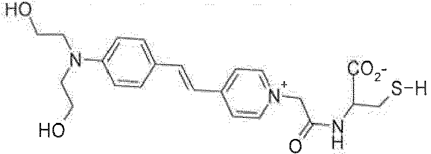 | **12** |
| 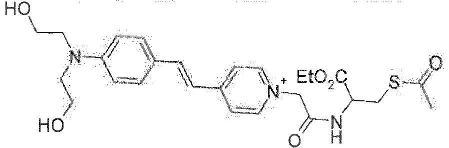 | **13** |
| 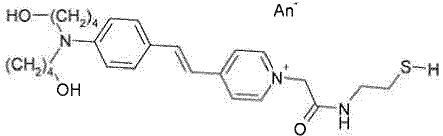 | **14** |
| 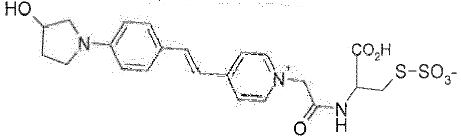 | **15** |
| 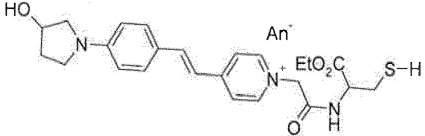 | **16** |
| 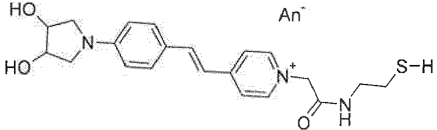 | **17** |
| 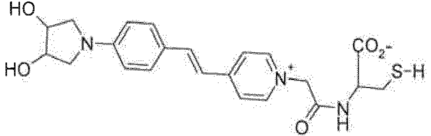 | **18** |
| 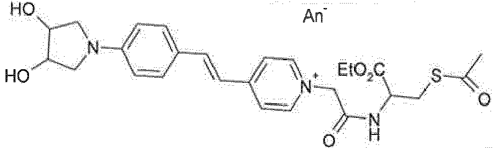 | **19** |
| 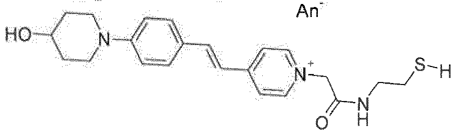 | **20** |
| 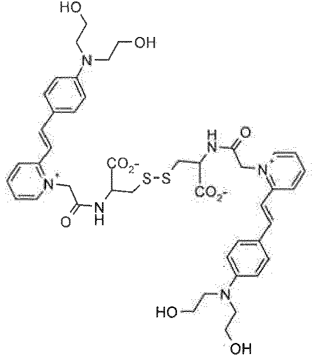 | **21** |
| 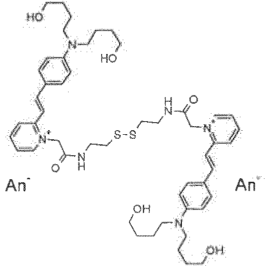 | **22** |
| 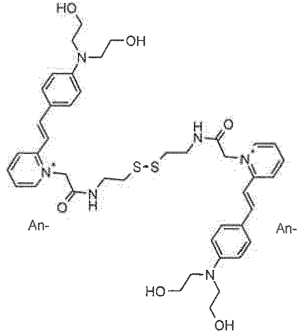 | **23** |
| 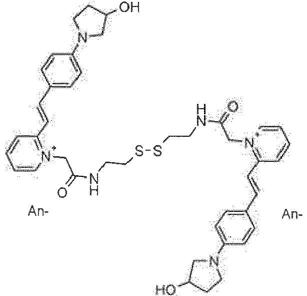 | **24** |
| 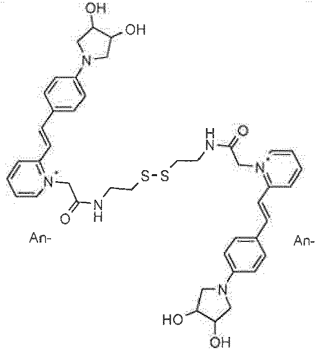 | **25** |
| 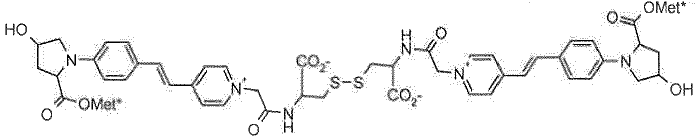 | **26** |
| 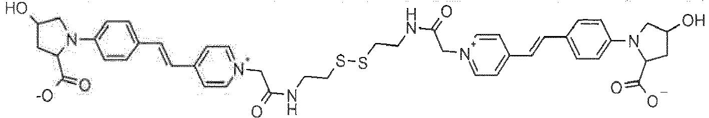 | **27** |
| 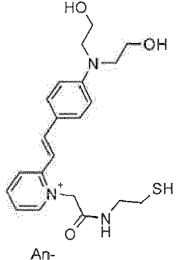 | **28** |
| 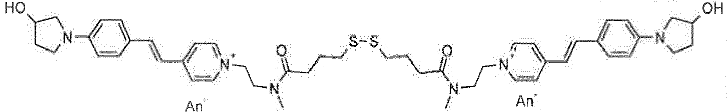 | **29** |
| 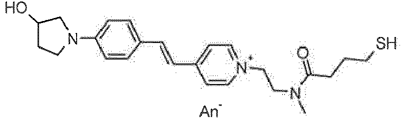 | **30** |
| 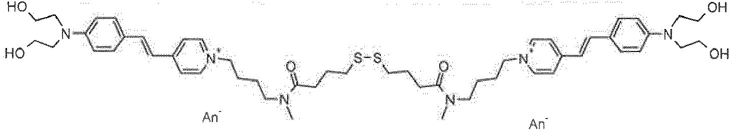 | **31** |
| 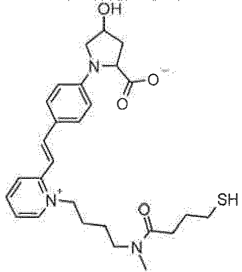 | **32** |
| 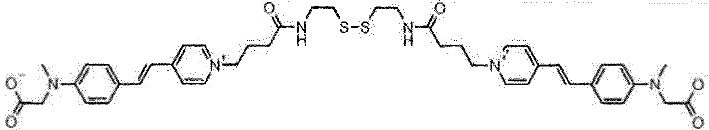 | **33** |
| 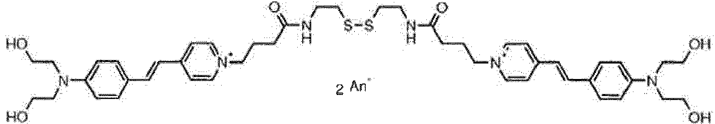 | **34** |
| 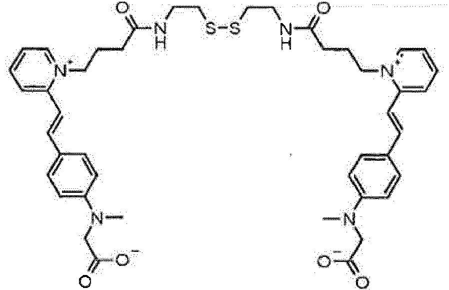 | **35** |
| 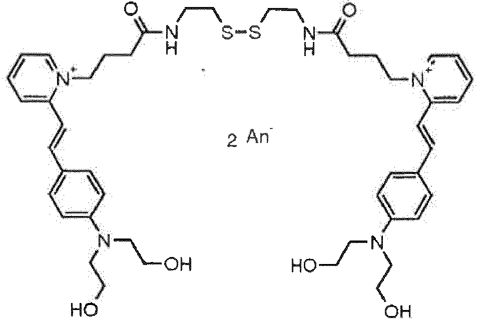 | **36** |
with An⁻ representing an anionic counterion.

9. Dye composition comprising, in a suitable cosmetic medium, a fluorescent dye of formula **(I)** as defined in any one of Claims 1 to 8.

10. Dye composition according to the preceding claim, also containing at least one reducing agent.

11. Process for dyeing keratin materials, in which a suitable dye composition comprising at least one fluorescent dye of formula (I) as defined in any one of Claims 1 to 8, optionally in the presence of a reducing agent, is applied to the materials.

12. Process for dyeing keratin materials according to the preceding claim, **characterized in that** the keratin materials are dark keratin fibres having a tone height of less than or equal to 6.

13. Multicompartment device in which a first compartment contains a dye composition comprising a fluorescent dye of formula **(I)** as defined in Claims 1 to 8, and a second compartment contains a reducing agent.

14. Use of the fluorescent dyes of formula **(I)** as defined in Claims 1 to 8, for dyeing human keratin fibres, in particular dark human keratin fibres particularly having a tone height of less than 6.

15. Use according to the preceding claim, for lightening dark keratin fibres particularly having a tone height of less than 6.
